# FASCICULE DE BREVET EUROPEEN

(11) **EP 2 566 839 B1**
(45) Date de publication et mention de la délivrance du brevet: **12.03.2014**
(21) Numéro de dépôt: 11731003.7
(22) Date de dépôt: 11.04.2011
(51) Int. Cl.: C07C 43/10

(54) **Composition de dérivés polyalcoxylés de triméthylolpropane et d'alcools gras procédé pour sa préparation et utilisation comme inverseur, dans les latex inverses auto-inversibles**
Zusammensetzungen aus polyalkoxylierten Derivaten von Trimethylolpropan und Fettalkoholen, Verfahren zur Herstellung dieser Zusammensetzung und ihre Verwendung als Umkehrmittel in selbstumkehrbaren Umkehrungslatexen
Composition made of polyalkoxylated derivatives of trimethylolpropane and fatty alcohols, method for preparing said composition, and use thereof as a reverser in self-reversible reverse latexes

(30) Priorité: 06.05.2010 FR 1053532
(43) Date de publication de la demande: 13.03.2013
(73) Titulaire: Société d'Exploitation de Produits pour les Industries Chimiques SEPPIC, 75007 Paris (FR)
(72) Inventeur: MALLO, Paul, F-78290 Croissy-sur-Seine (FR); ROLLAND, Hervé, F-81100 Castres (FR); DA COSTA, Georges, F-81710 Saix (FR); BRAUN, Olivier, F-81100 Castres (FR)
(74) Mandataire: Conan, Philippe Claude
(86) Numéro de dépôt international: PCT/FR2011/050817
(87) Numéro de publication internationale: WO 2011/138534

(56) Documents cités:
- FR-A- 2 467 186
- FR-A- 2 879 607
- US-A1- 2005 131 205
- XIAOFEN HU, JIAN JI: "Construction of Multifunctional Coatings via Layer-by-Layer Assembly ofSulfonated Hyperbranched Polyether and Chitosan", LANGMUIR, vol. 26, no. 4, 18 septembre 2009 (2009-09-18), pages 2624-2629, XP002600733,

## Description

La présente demande concerne l'utilisation de composés dérivés du triméthylolpropane oxétane comme inverseurs dans les latex inverses auto-inversibles.

Les latex inverses de polyélectrolytes à base de l'acide 2-méthyl 2-[(1-oxo 2-propènyl) amino] 1-propanesulfonique (dénommé aussi acide 2-acrylamido 2-méthyl propanesulfonique, ATBS ou AMPS) partiellement ou totalement salifié ainsi que leur utilisation en cosmétique et/ou en pharmacie ont fait l'objet de nombreuses demandes de brevet. Cependant, la présence de quantités importantes d'eau et d'huile dans ces latex inverses représente un inconvénient non négligeable en termes de volume, de coût et parfois de risques accrus et/ou d'effets toxiques.

Des solutions ont donc été développées pour augmenter la concentration en polyélectrolytes dans les latex inverses finaux par exemple en soumettant le milieu réactionnel, en fin de polymérisation, à une étape de distillation sous vide pour enlever une partie plus ou moins importante d'eau et d'huile. Cette distillation est cependant délicate à mettre en oeuvre car elle induit souvent une déstabilisation du latex inverse qu'il faut contrer par l'addition préalable d'agents stabilisants.

Les demandes de brevet européen EP 0 161 038 et EP 0 126 528 ainsi que la demande de brevet britannique GB 1 482 515 divulguent une telle utilisation de polymères stabilisants.

L'inconvénient de ces produits stabilisants est qu'ils contiennent des alcools ou des glycols pouvant induire des problèmes environnementaux. De plus il se produit parfois une prise en masse du milieu réactionnel lors de l'étape de distillation sans que ce phénomène n'ait jamais vraiment été expliqué, mais dont la conséquence certaine est la destruction du lot de latex inverse en cours de préparation et un nettoyage pénible et coûteux du réacteur utilisé. Enfin, même quand la distillation se déroule correctement, les latex inverses obtenus s'inversent souvent difficilement lors de leur mise en oeuvre dans une phase aqueuse, et ils présentent aussi une viscosité élevée et parfois en leur sein des micro-gels. Ces inconvénients interdisent donc leur utilisation dans la fabrication de formulations cosmétiques et/ou de pâtes d'impression textile. Pour pallier à ces inconvénients, les inventeurs ont développé un latex inverse divulgué dans la demande de brevet français publiée sous le numéro FR 2 879 607 comprenant de 50% à 80% massique d'un polyélectrolyte comportant de 0,01 % à 10 % molaire d'au moins une unité monomérique dérivée du composé de formule (A) :

C(R₁)(R₃)=C(R₂)-C(=O)-O-(CH₂-CH₂-O)ₙ-R₄ (A)

dans laquelle les radicaux R₁, R₂ et R₃, identiques ou différents représentent indépendamment les uns des autres un atome d'hydrogène ou un radical alkyle linéaire ou ramifié comportant de 1 à 4 atomes de carbone, le radical R4 représente un radical aliphatique linéaire ou ramifié, saturé ou insaturé, comportant de 6 à 30 atomes de carbone et n représente un nombre compris entre 1 et 50.

Cependant, lorsque l'on met en oeuvre cette composition pour préparer une formulation épaissie, la vitesse d'inversion du latex inverse dans la phase aqueuse, c'est-à-dire le temps nécessaire pour obtenir le développement maximal de la viscosité reste assez faible ce qui signifie pour l'utilisateur une perte de temps lors de l'utilisation de ce produit surtout en phase industrielle pour préparer des formulations cosmétiques et/ou des pâtes d'impression textile. En effet il est bien connu que le temps d'inversion des latex inverses augmente très considérablement en fonction de l'échelle d'utilisation. Par ailleurs la stabilité dans le temps des latex inverses décrits dans FR 2 879 607 n'est pas totalement satisfaisante .On observe en effet un phénomène de relargage d'huile en surface assez rapide pendant le stockage.

Les inventeurs ont donc cherché à développer des latex inverses qui ne présentent pas les inconvénients précités.

Selon un premier aspect, l'invention a pour objet **une composition tensioactive (C),** qui comprend pour 100% molaire :
1) - Une proportion supérieure ou égale à 10% molaire et inférieure ou égale à 50% molaire d'une composition (C_{II}) comprenant pour 100% molaire :
   α) - De 60% molaire à 100% molaire d'un composé de formule (II) : 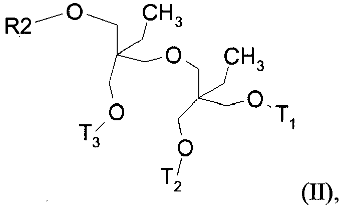 dans laquelle :
      - R2 représente un radical alkyle linéaire ou ramifié, comportant de 12 atomes de carbone,
      - T₁, représente un atome d'hydrogène ou un radical (-CH₂-CH₂-O-)ₘ₁-H dans lequel m1 est un nombre entier supérieur ou égal à zéro et inférieur ou égal à dix,
      - T₂ identique ou différent de T₁, représente un atome d'hydrogène ou un radical (-CH₂-CH₂-O-)ₘ₂-H dans lequel m2 est un nombre entier supérieur ou égal à zéro et inférieur ou égal à dix, et
      - T₃ identique ou différent de T₁ et de T₂, représente un atome d'hydrogène ou un radical (-CH₂-CH₂-O-)ₘ₃-H dans lequel m3 est un nombre entier supérieur ou égal à zéro et inférieur ou égal à dix,
      - étant entendu que la somme m1 + m2 + m3 est supérieure à 0 et inférieure ou égale à dix ;
   β) - Optionnellement jusqu'à 40% molaire d'un composé de formule (II') 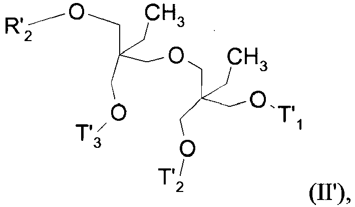 dans laquelle :
      - R'₂ représente un radical alkyle linéaire ou ramifié, comportant 14 atomes de carbone,
      - T'₁, représente un atome d'hydrogène ou un radical (-CH₂-CH₂-O-)ₘ₁-H dans lequel m1 est un nombre entier supérieur ou égal à zéro et inférieur ou égal à dix,
      - T'₂ identique ou différent de T'₁, représente un atome d'hydrogène ou un radical (-CH₂-CH₂-O-)Cₘ₂-H dans lequel m2 est un nombre entier supérieur ou égal à zéro et inférieur ou égal à dix, et
      - T'₃ identique ou différent de T'₁ et de T'₂, représente un atome d'hydrogène ou un radical (-CH₂-CH₂-O-)Cₘ₃-H dans lequel m3 est un nombre entier supérieur ou égal à zéro et inférieur ou égal à dix,
      - étant entendu, que la somme m1 + m2 + m3 est supérieure à 0 et inférieure ou égale à dix ; et
   γ) - Optionnellement jusqu'à 10% molaire d'un composé de formule (II") : 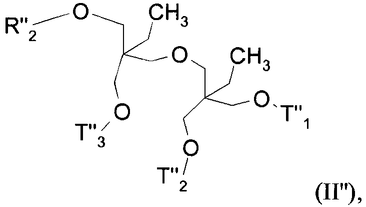 dans laquelle :
      - R"₂ représente un radical alkyle linéaire ou ramifié, comportant 16 atomes de carbone,
      - T"₁, représente un atome d'hydrogène ou un radical (-CH₂-CH₂-O-)ₘ₁-H dans lequel m1 est un nombre entier supérieur ou égal à zéro et inférieur ou égal à dix,
      - T"₂ identique ou différent de T"₁, représente un atome d'hydrogène ou un radical (-CH₂-CH₂-O-)ₘ₂-H dans lequel m2 est un nombre entier supérieur ou égal à zéro et inférieur ou égal à dix, et
      - T"₃ identique ou différent de T"₁ et de T"₂, représente un atome d'hydrogène ou un radical (-CH₂-CH₂-O-)ₘ₃-H dans lequel m3 est un nombre entier supérieur ou égal à zéro et inférieur ou égal à dix,
      - étant entendu que la somme m1 + m2 + m3 est supérieure à 0 et inférieure ou égale à dix ;
2) - Une proportion supérieure ou égale à 50% molaire et inférieure ou égale à 90% molaire d'une composition (C_{III}) comprenant pour 100% molaire :
   α) - De 60% molaire à 100% molaire d'un composé de formule (III) : 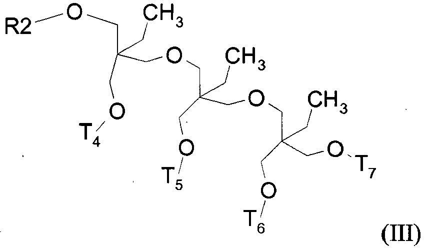 ou de son isomère de formule (IV) : 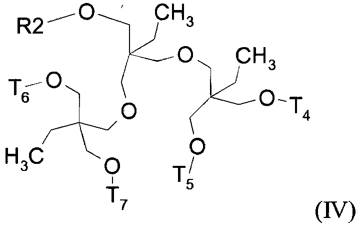 ou du mélange de ces deux isomères,
      formules (III) et (IV) dans lesquelles :
      - R2 représente un radical alkyle linéaire ou ramifié, comportant 12 atomes de carbone,
      - T₄, représente un atome d'hydrogène ou un radical (-CH₂-CH₂-O-)ₘ₄-H dans lequel m4 est un nombre entier supérieur ou égal à zéro et inférieur ou égal à dix,
      - T₅ identique ou différent de T₄, représente un atome d'hydrogène ou un radical (-CH₂-CH₂-O-)ₘ₅-H dans lequel m5 est un nombre entier supérieur ou égal à zéro et inférieur ou égal à dix,
      - T₆ identique ou différent de T₄ et de T₅ représente un atome d'hydrogène ou un radical (-CH₂-CH₂-O-)ₘ₆-H dans lequel m6 est un nombre entier supérieur ou égal à zéro et inférieur ou égal à dix,
      - T₇ identique ou différent de T₄ de T₅ et de T₆, représente un atome d'hydrogène ou un radical (-CH₂-CH₂-O-)ₘ₇-H dans lequel m7 est un nombre entier supérieur ou égal à zéro et inférieur ou égal à dix étant entendu que la somme m4 + m5 + m6 + m7 est supérieure à 0 et inférieure ou égale à dix ;
   P) - Optionnellement jusqu'à 40% molaire composé de formule (III') : 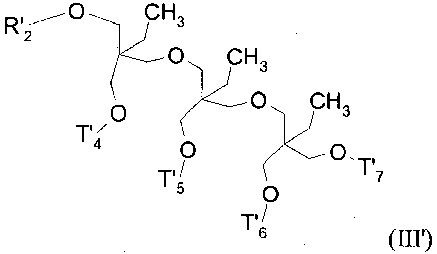 ou de son isomère de formule (IV') : 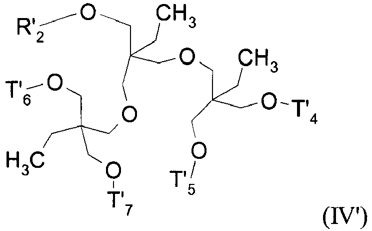 ou du mélange de ces deux isomères,
      formules (III') et (IV') dans lesquelles :
      - R'₂ représente un radical alkyle linéaire ou ramifié, comportant 14 atomes de carbone,
      - T'₄, représente un atome d'hydrogène ou un radical (-CH₂-CH₂-O-)ₘ₄-H dans lequel m4 est un nombre entier supérieur ou égal à zéro et inférieur ou égal à dix,
      - T'₅ identique ou différent de T'₄, représente un atome d'hydrogène ou un radical (-CH₂-CH₂-O-)ₘ₅-H dans lequel m5 est un nombre entier supérieur ou égal à zéro et inférieur ou égal à dix,
      - T'₆ identique ou différent de T'₄ et de T'₅, représente un atome d'hydrogène ou un radical (-CH₂-CH₂-O-)ₘ₆-H dans lequel m6 est un nombre entier supérieur ou égal à zéro et inférieur ou égal à dix, et
      - T'₇ identique ou différent de T'₄, de T'₅ et de T'₆, représente un atome d'hydrogène ou un radical (-CH₂-CH₂-O-)ₘ₇-H dans lequel m7 est un nombre entier supérieur ou égal à zéro et inférieur ou égal à dix,
      - étant entendu que la somme m4 + m5 + m6 + m7 est supérieure à 0 et inférieure ou égale à dix ; et
   γ) - Optionnellement jusqu'à 10% molaire d'un composé de formule (III") : 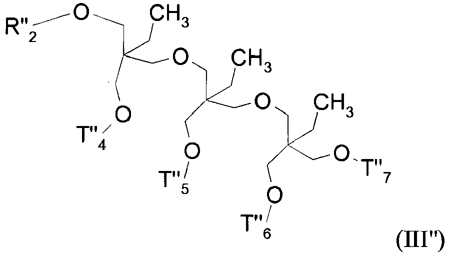 ou de son isomère de formule (IV") : 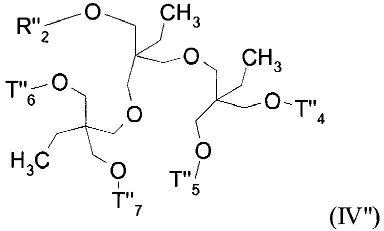 ou du mélange de ces deux isomères,
      formules (III") et (IV") dans lesquelles :
      - R"₂ représente un radical alkyle linéaire ou ramifié, comportant 16 atomes de carbone,
      - T"₄, représente un atome d'hydrogène ou un radical (-CH₂-CH₂-O-)ₘ₄-H dans lequel m4 est un nombre entier supérieur ou égal à zéro et inférieur ou égal à dix,
      - T"₅ identique ou différent de T"₄, représente un atome d'hydrogène ou un radical (-CH₂-CH₂-O-)ₘ₅-H dans lequel m5 est un nombre entier supérieur ou égal à zéro et inférieur ou égal à dix,
      - T"₆ identique ou différent de T"₄ et de T"₅ représente un atome d'hydrogène ou un radical (-CH₂-CH₂-O-)ₘ₆-H dans lequel m6 est un nombre entier supérieur ou égal à zéro et inférieur ou égal à dix, et
      - T"₇ identique ou différent de T"₄, de T"₅ et de T"₆, représente un atome d'hydrogène ou un radical (-CH₂-CH₂-O-)ₘ₇-H dans lequel m7 est un nombre entier supérieur ou égal à zéro et inférieur ou égal à dix,
      - étant entendu que la somme m4 + m5 + m6 + m7 est supérieure à 0 et inférieure ou égale à dix ; Selon un autre aspect particulier de la présente invention, ladite composition tensioactive (C) telle que définie précédemment, **comprend en outre :**
3) - Jusqu'à 5% molaire d'une composition (C_{V}) comprenant pour 100% molaire
   α) - De 60% molaire à 100% molaire d'un composé de formule (V) : 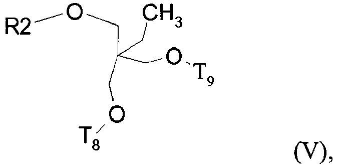 dans laquelle :
      - R2 représente un radical alkyle linéaire ou ramifié, comportant de 12 atomes de carbone,
      - T₈, représente un atome d'hydrogène ou un radical (-CH₂-CH₂-O-)ₘ₈-H dans lequel m8 est un nombre entier supérieur ou égal à zéro et inférieur ou égal à dix,
      - T₉ identique ou différent de T₈, représente un atome d'hydrogène ou un radical (-CH₂-CH₂-O-)Cₘ₉-H dans lequel m9 est un nombre entier supérieur ou égal à zéro et inférieur ou égal à dix, et
      - étant entendu que la somme m8 + m9 est supérieure à 0 et inférieure ou égale à dix ;
   P) - Optionnellement jusqu'à 40% molaire d'un composé de formule (V') 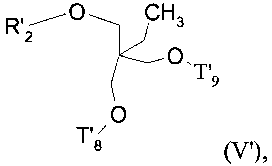 dans laquelle :
      - R'₂ représente un radical alkyle linéaire ou ramifié, comportant 14 atomes de carbone,
      - T'₈, représente un atome d'hydrogène ou un radical (-CH₂-CH₂-O-)ₘ₈-H dans lequel m8 est un nombre entier supérieur ou égal à zéro et inférieur ou égal à dix,
      - T'₉ identique ou différent de T'₈, représente un atome d'hydrogène ou un radical (-CH₂-CH₂-O-)ₘ₉-H dans lequel m9 est un nombre entier supérieur ou égal à zéro et inférieur ou égal à dix, et
      - étant entendu que la somme m8 + m9 est supérieure à 0 et inférieure ou égale à dix ; et
   γ) - Optionnellement jusqu'à 10% molaire d'un composé de formule (V") : 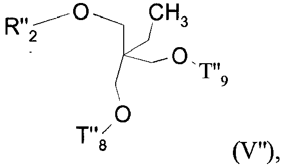 dans laquelle :
      - R"₂ représente un radical alkyle linéaire ou ramifié, comportant 16 atomes de carbone,
      - T"₈, représente un atome d'hydrogène ou un radical (-CH₂-CH₂-O-)ₘ₈-H dans lequel m8 est un nombre entier supérieur ou égal à zéro et inférieur ou égal à dix,
      - T"₉ identique ou différent de T"₈, représente un atome d'hydrogène ou un radical (-CH₂-CH₂-O-)ₘ₉-H dans lequel m9 est un nombre entier supérieur ou égal à zéro et inférieur ou égal à dix, et
      - étant entendu que la somme m8 + m9 est supérieure à 0 et inférieure ou égale à dix.
      Selon un autre aspect particulier de la présente invention, ladite composition active (C) telle que définie précédemment, **comprend en outre :**
4) - Jusqu'à 5% molaire d'une composition (C_{VI}) comprenant pour 100% molaire :
   α) - De 60% molaire à 100% molaire d'un composé de formule (VI) :

      R2-(-CH₂-CH₂-O-)ₘ₁₀-H (VI),

      dans laquelle R2 représente un radical alkyle linéaire ou ramifié, comportant 12 atomes de carbone et m10 est un nombre entier supérieur ou égal à zéro et inférieur ou égal à dix ;
   β) - Optionnellement jusqu'à 40% molaire d'un composé de formule (VI') :

      R'₂-(-CH₂-CH₂-O-)ₘ₁₀-H (VI'),

      dans laquelle R'₂ représente un radical alkyle linéaire ou ramifié, comportant 14 atomes de carbone et m10 est un nombre entier supérieur ou égal à zéro et inférieur ou égal à dix ; et
   γ) - Optionnellement jusqu'à 10% molaire d'un composé de formule (VI") :

      R"₂-(-CH₂-CH₂-O-)ₘ₁₀-H (VI"),

      dans laquelle R"₂ représente un radical alkyle linéaire ou ramifié, comportant 16 atomes de carbone et m10 est un nombre entier supérieur ou égal à zéro et inférieur ou égal à dix.

Selon un aspect particulier de la présente invention, **la dite composition tensioactive (C)** telle que définie précédemment, comprend :
1) - Une proportion supérieure ou égale à 20% molaire et inférieure ou égale à 50% molaire d'une composition (C_{II}) telle que définie précédemment ;
2) - Une proportion supérieure ou égale à 50% molaire et inférieure ou égale à 80% molaire d'une composition (C_{III}) telle que définie précédemment.
telle que définie précédemment

Selon un autre aspect particulier de la présente invention :
1) - ladite composition (C_{II}) comprend pour 100% molaire :
   α) - De 60% à 80% molaire du composé de formule (II),
   β) - De 15% à 30% molaire du composé de formule (II'), et
   γ) - Jusqu'à 10% molaire du composé de formule (II"), et
2) - ladite composition (C_{III}) comprend pour 100% molaire :
   α) - De 60% à 80% molaire du composé de formule (III), de son isomère de formule (IV) ou du mélange de ces isomères,
   β) - de 15% à 30% molaire du composé de formule (III'), de son isomère de formule (IV') ou du mélange de ces isomères, et
   γ) - Jusqu'à 10% molaire du composé de formule (III"), de son isomère de formule (IV") ou du mélange de ces isomères.

L'invention a aussi pour objet un procédé de préparation de ladite composition tensioactive (C) telle que définie précédemment, caractérisé en ce qu'il comprend les étapes successive suivantes :
**Une étape a)** de réaction d'un mélange d'alcools comprenant pour 100% molaire :
   **de 60% molaire à 100% molaire d'un composé de formule (VII),**

      R2-O-H (VII),

      dans laquelle R2 représente un radical alkyle linéaire ou ramifié, comportant 12 atomes de carbone;
   **optionnellement jusqu'à 40% molaire d'un composé de formule (VII') :**

      R'₂-O-H (VII'),

      dans laquelle R'₂ représente un radical alkyle linéaire ou ramifié, comportant 14 atomes de carbone, et
   **optionnellement jusqu'à 10% molaire d'un composé de formule (VI") :**

      R"₂-O-H (VII"),

      dans laquelle R"₂ représente un radical alkyle linéaire ou ramifié, comportant 16 atomes de carbone;
      avec un excès stoechiométrique de 3-(hydroxyméthyl) 3-éthyl oxétane de formule (VIII) : 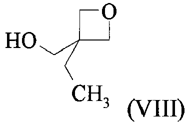 pour former une composition (C') comprenant le composé de formule (IX) : 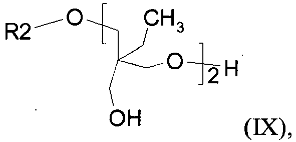 dans laquelle R2 est tel que défini ci-dessus ;
      optionnellement le composé de formule (IX)' : 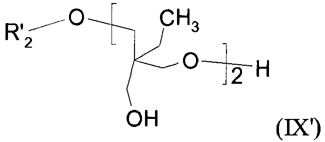 dans laquelle R'₂ est tel que défini ci-dessus ;
      optionnellement le composé de formule (IX") : 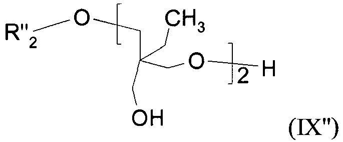 dans laquelle R"₂ est tel que défini ci-dessus ;
      le composé de formule (X) : 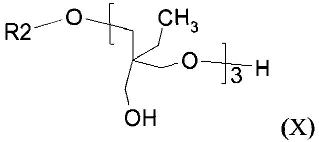 ou son isomère de formule (XI) : 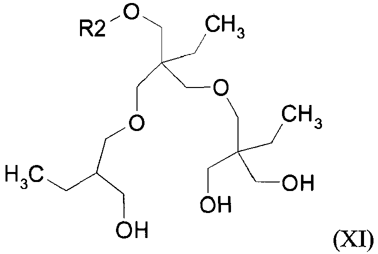 ou un mélange de ces deux isomères ; composés de formules (X) et (XI) dans lesquelles R2 est tel que défini ci-dessus ;
      optionnellement le composé de formule (X') : 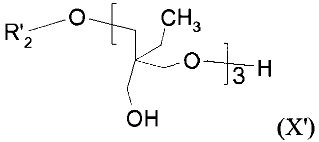 ou son isomère de formule (XI') : 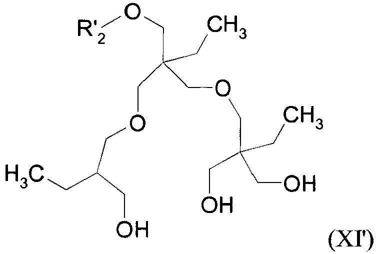 ou un mélange de ces deux isomères ; composés de formules (X') et (XI') dans lesquelles R'₂ est tel que défini ci-dessus ;
      optionnellement le composé de formule (X") : 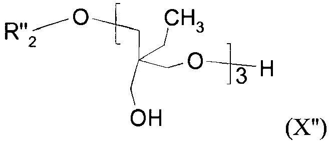 ou son isomère de formule (XI") : 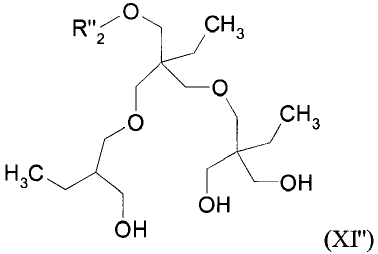 ou un mélange de ces deux isomères ; composés de formules (X") et (XI") dans lesquelles R"₂ est tel que défini ci-dessus ;
**Une étape b)** de réaction dans le rapport stoechiométrique souhaité, de ladite composition (C') avec l'oxyde d'éthylène de formule (XII): 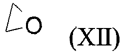 pour former ladite composition tensioactive (C).

L'invention a aussi pour objet la composition C', intermédiaire du procédé tel que défini précédemment.

L'invention a encore pour objet, les composés de formules (IX), (X) et (XI), intermédiaires du procédé tel que défini précédemment.

L'invention a enfin pour objet l'utilisation **de la composition tensioactive (C)**, telle que définie précédemment, comme agent émulsionnant de type huile dans eau (H/E).et plus particulièrement comme agent émulsionnant de type huile dans eau (H/E), plus particulièrement comme agent inverseur d'un latex inverse auto-inversible, seul ou mélange avec d'autres agent émulsionnants aptes et destinés à préparer un système émulsionnant (S₂) de type huile dans eau (H/E),

Par latex inverse auto-inversible, on désigne une émulsion eau dans huile d'un polymère réticulé, obtenue par polymérisation en émulsion inverse des monomères mis en oeuvre, ladite émulsion eau dans huile d'un polymère étant apte grâce à la présence en son sein d'un système émulsionnant (S₂) de type huile dans eau (H/E), dit agent inverseur, à s'inverser en émulsion huile dans eau par simple dispersion dans l'eau sous agitation mécanique lente, provoquant ainsi l'épaississement de cette phase aqueuse.

Comme exemple de latex inverse auto-inversibles pouvant comprendre avantageusement la composition tensioactive (C) telle que définie précédemment, au sein du (S₂) de type huile dans eau (H/E), il y a par exemple, une composition comprenant pour 100% massique :
a) de 20 % massique à 80 % massique d'un polyélectrolyte anionique (P) réticulé et/ou branché, obtenu par polymérisation :
   - d'au moins un monomère neutre choisi notamment parmi l'acrylamide, le N,N-diméthyl acrylamide, le N-[2-hydroxy-1,1-bis(hydroxyméthyl) éthyl] propènamide [ou tris(hydroxyméthyl) acrylamidométhane ou N-tris(hydroxyméthyl) méthyl acrylamide dénommé aussi THAM] ou l'acrylate de (2-hydroxy éthyle) ;
      et/ou
      - d'au moins un monomère comportant une fonction acide fort ;
      et/ou
      - d'au moins un monomère comportant une fonction acide faible ; et/ou
      - d'au moins un monomère neutre de formule (I) : 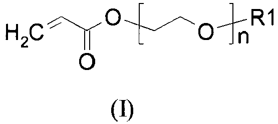
      dans laquelle le radical R1 représente un radical aliphatique linéaire ou ramifié, comportant de 8 à 20 atomes de carbone et n représente un nombre supérieur ou égal à un et inférieur ou égal à trente ;
b) de 5 % massique à 10 % massique d'un système émulsionnant (S₁) de type eau dans huile (E/H) ;
c) de 1 % massique à 10% massique d'un système émulsionnant (S₂) de type huile dans eau (H/E) comprenant une proportion massique non nulle de ladite composition tensioactive (C) telle que définie précédemment ;
d) de 5 % massique à 45 % massique d'au moins une huile, et
e) de 0 % massique à 45 % massique d'eau.

Par radical aliphatique linéaire ou ramifié, saturé ou insaturé, comportant de 6 à 20 atomes de carbone, on désigne pour le radical R1 dans la formule (I) telle que définie ci-dessus, plus particulièrement les radicaux linéaires tels que, par exemple, les radicaux, hexyle, octyle, nonyle, décyle, undécyle, dodécyle, tétradécyle, hexadécyle, octadécyle ou eicosyle.

Dans la composition telle que définie ci-dessus, le système émulsionnant (S₁) de type eau dans huile (E/H) est constitué soit d'un seul tensioactif soit d'un mélange de tensioactifs à condition que ledit tensioactif ou ledit mélange ait une valeur de HLB suffisamment faible pour induire des émulsions eau dans huile. Comme agent émulsionnant de type eau dans huile, il y a par exemple les esters de sorbitan, comme l'oléate de sorbitan, comme celui commercialisé par la société SEPPIC sous le nom MONTANE^{™} 80, l'isostéarate de sorbitan, comme celui commercialisé par la société SEPPIC sous le nom MONTANE™ 70 ou le sesquioléate de sorbitan comme celui commercialisé par la société SEPPIC sous le nom MONTANE™ 83. Il y aussi certains esters de sorbitan polyéthoxylés, par exemple le monoléate de sorbitan pentaéthoxylé comme celui commercialisé par la société SEPPIC sous le nom MONTANOX™ 81 ou l'isostéarate de sorbitan pentaéthoxylé comme celui commercialisé sous le nom MONTA-NOX™ 71 par la société SEPPIC. Il y a encore l'alcool oléocétylique diéthoxylé comme celui commercialisé sous le nom SIMULSOL™ OC 72 par la société SEPPIC, les polyesters de poids moléculaire compris entre 1000 et 3000, produits de la condensation entre un acide poly(isobutènyl) succinique ou son anhydride et tels que l'HYPERMER™ 2296 commercialisé par la société UNIQEMA ou enfin les copolymères blocks de poids moléculaire compris entre 2500 et 3500, comme l'HYPERMER™ B246 commercialisé par la société UNIQEMA ou le SIMALINE™ IE 200 commercialisé par la société SEPPIC.

Dans le cadre de cette utilisation la composition tensioactive (C) telle que définie précédemment peut être mise en oeuvre seule ou en mélange avec au moins un autre agent émulsionnant de type huile dans eau (H/E), au sein dudit système émulsionnant (S₂) de type huile dans eau (H/E), agent inverseur de latex inverses auto-inversibles.

Selon un mode particulier des latex inverses auto-inversibles tels que définis précédemment, le système émulsionnant (S₂) de type huile dans eau (H/E) consiste en 100% massique de ladite composition tensioactive (C) telle que définie précédemment.

Selon un autre mode particulier des latex inverses auto-inversibles tels que définis précédemment, le système émulsionnant (S₂) de type huile dans eau (H/E) comprend en outre au moins un tensioactif émulsionnant du type (H/E) différent de l'un ou de l'autre des composés constituant ladite composition tensioactive (C) telle que définies précédemment.

Par "agent émulsionnant du type huile dans eau", on désigne des agents émulsionnants possédant une valeur de HLB suffisamment élevée pour fournir des émulsions huile dans l'eau tels que les esters de sorbitan éthoxylés comme l'oléate de sorbitan polyéthoxylé avec 20 moles d'oxyde d'éthylène, commercialisé par la société SEPPIC sous le nom de MONTANOX^{™} 80, le laurate de sorbitan polyéthoxylé avec 20 moles d'oxyde d'éthylène, commercialisé par la société SEPPIC sous le nom de MONTANOX^{™} 20, l'huile de ricin polyéthoxylée avec 40 moles d'oxyde d'éthylène commercialisé sous le nom SIMULSOL^{™} OL50, l'alcool oléodécylique décaéthoxylé, commercialisé par la société SEPPIC sous le nom SIMULSOL^{™} OC 710, l'alcool laurique heptaéthoxylé commercialisé sous le nom SIMULSOL^{™} P7, le nonylphénol décaéthoxylé commercialisé par la société SEPPIC sous le nom NONAROX^{™}-10-30 ou les hexaoléates de sorbitan polyéthoxylés commercialisés par la société SEPPIC sous le nom SI-MALINE^{™} IE 400.

Selon un mode plus particulier des latex inverses auto-inversibles tels que définis précédemment, le système émulsionnant (S₂) de type huile dans eau (H/E) comprend en outre une proportion massique non nulle d'au moins un agent émulsionnant du type huile dans eau choisi parmi l'oléate de sorbitan polyéthoxylé avec 20 moles d'oxyde d'éthylène, le laurate de sorbitan polyéthoxylé avec 20 moles d'oxyde d'éthylène, l'huile de ricin polyéthoxylée avec 40 moles d'oxyde d'éthylène, l'alcool oléodécylique décaéthoxylé, l'alcool laurique heptaéthoxylé le nonylphénol décaéthoxylé ou les hexaoléates de sorbitan polyéthoxylés Selon ce mode particulier ledit système émulsionnant (S₂) comprend au moins 30% massique dudit mélange (M) tel que défini précédemment.

Selon un mode tout particulier des latex inverses auto-inversibles tels que définis précédemment, le système émulsionnant (S₂) de type huile dans eau comprend pour 100% de sa masse :
- de 10% massique à 40% massique d'alcool laurique heptaéthoxylé et
- de 60% massique à 90% massique de la composition tensioactive (C) telle que définie précédemment.

Par polyélectrolyte réticulé on désigne pour (P), un polyélectrolyte non linéaire se présentant à l'état de réseau tridimensionnel insoluble dans l'eau, mais gonflable à l'eau et conduisant donc à l'obtention d'un gel chimique.

Par polyélectrolyte branché, on désigne pour (P), un polymère non linéaire qui possède des chaînes pendantes de manière à obtenir, lorsque ce polymère est mis en solution dans l'eau, un fort état d'enchevêtrement conduisant à des viscosités à bas gradient très importantes.

Le polyélectrolyte (P) est plus particulièrement réticulé avec un composé diéthylénique ou polyéthylènique dans la proportion molaire exprimée par rapport aux monomères mis en oeuvre généralement inférieure ou égale à 0,40% molaire, principalement inférieure à 0,25% molaire, plus particulièrement inférieure ou égale à 0,05 % molaire et tout particulièrement comprise entre 0,005 % et 0,01 %.molaire. De préférence, l'agent de réticulation et/ou l'agent de ramification est choisi parmi le diméthacrylate d'éthylèneglycol, le diacrylate de diéthylèneglycol, le diallyloxyacétate de sodium, le diacrylate d'éthylèneglycol, le diallyl urée, le triallylamine, le triméthylol propanetriacrylate, le méthylène-bis-(acrylamide) ou un mélange de ces composés.

Comme exemples de polyélectrolytes réticulés (P) constitutifs desdits latex inverses auto-inversibles il y a :
- Les terpolymères réticulés de l'acide acrylique partiellement salifié sous forme de sel de sodium ou de sel d'ammonium, de l'acrylamide et de l'acrylate de lauryle tétraéthoxylé ;
- Les terpolymères réticulés de l'acide 2-méthyl 2-[(1-oxo 2-propènyl) amino] 1-propanesulfonique partiellement salifié sous forme de sel de sodium, de l'acrylamide et de l'acrylate de lauryle tétraéthoxylé ;
- Les terpolymères réticulés de l'acide 2-méthyl 2-[(1-oxo 2-propènyl) amino] 1-propanesulfonique, de l'acide acrylique partiellement salifiés sous forme de sel de sodium et de l'acrylate de lauryle tétraéthoxylé ;
- Les terpolymères réticulés de l'acide 2-méthyl 2-[(1-oxo 2-propènyl) amino] 1-propanesulfonique partiellement salifié sous forme de sel de sodium, de l'acrylate de 2-hydroxy éthyle et de l'acrylate de lauryle tétraéthoxylé ;
- Les copolymères réticulés de l'acide 2-méthyl 2-[(1-oxo 2-propènyl) amino] 1-propanesulfonique partiellement salifié sous forme de sel de sodium et de l'acrylate de lauryle tétraéthoxylé ;
- Les copolymères réticulés de l'acide acrylique partiellement salifié sous forme de sel d'ammonium ou de sel de monoéthanolamine et de l'acrylate de lauryle tétraéthoxylé ;
- Les tétrapolymères réticulés de l'acrylamide, de l'acide 2-méthyl 2-[(1-oxo 2-propènyl) amino] 1-propanesulfonique, de l'acide acrylique partiellement salifiés sous forme de sel sodium et de l'acrylate de lauryle tétraéthoxylé ; et
- Les tétrapolymères de l'acide 2-méthyl 2-[(1-oxo 2-propènyl) amino] 1-propanesulfonique partiellement salifié sous forme de sel de sodium, de l'acrylamide, du vinyl pyrrolidone et de l'acrylate de lauryle tétraéthoxylé ;
- Les pentapolymères réticulés de l'acide 2-méthyl 2-[(1-oxo 2propènyl) amino] 1-propanesulfonique partiellement ou totalement salifié sous forme de sel de sodium, de l'acide acrylique partiellement salifié sous forme de sel sodium, de l'acrylate de 2-hydroxy éthyle, de tris(hydroxyméthyl)aminométhyl acrylamide et de l'acrylate de lauryle tétraéthoxylé. Le polyélectrolyte anionique (P) réticulé constitutif desdits latex inverses auto-inversibles, comporte pour 100% de monomères mis en oeuvre plus particulièrement :
   - De 20% molaire à 80% molaire d'unités monomériques issues d'un monomère comportant soit une fonction acide fort, soit une fonction acide faible ;
   - De 15% molaire à 75% molaire d'unités monomériques issues d'un monomère neutre différent du composé de formule (I) telle que définie précédemment ;
   - De 0,5% à 5% molaire d'unités monomériques issues d'un monomère de formule (I) telle que définie précédemment,

Selon un autre aspect particulier, le polyélectrolyte anionique (P) réticulé comporte pour 100% de monomères mis en oeuvre :
- De 55% molaire à 80% molaire d'unités monomériques issues d'un monomère comportant une fonction acide fort ;
- De 15% molaire à 40% molaire d'unités monomériques issues d'un monomère neutre différent du composé de formule (I) telle que définie précédemment ;
- De 1% à 5% molaire d'unités monomériques issues d'un monomère de formule (I) telle que définie précédemment,

Selon un autre mode particulier, ledit latex inverse auto-inversible tell que défini précédemment, comprend plus de 60 % en poids et au plus 70 % en poids de polyélectrolyte anionique (P).

Dans lesdits latex inverses auto-inversibles tels que définis précédemment, la phase huile est constituée soit par une huile minérale commerciale contenant des hydrocarbures saturés comme les paraffines, les isoparaffines, les cycloparaffines, présentant à température ambiante, une densité entre 0,7 et 0,9 et un point d'ébullition supérieur à environ 250°C, telle que par exemple le MARCOL^{™}52 commercialisés par EXXON CHEMICAL, soit par une huile végétale comme le squalane d'origine végétale, soit une huile de synthèse tel que le polyisobutène hydrogéné ou le polydécène hydrogéné, soit par un mélange de plusieurs de ces huiles. Le MARCOL^{™} 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993). La composition telle que définie précédemment peut également contenir divers additifs tels que des agents complexants, des agents de transfert ou des agents limiteurs de chaîne.

Les exemples qui suivent ont pour but d'illustrer la présente invention sans toutefois la limiter.

### Exemple A) : Préparation d'une composition tensioactive (C) mise en oeuvre dans la composition objet de la présente invention

### Stade A1) : Préparation de la composition intermédiaire (C')

21.100 g d'un mélange d'alcools gras comprenant de 65% à 75% massique d'alcanol comportant 12 atomes de carbone, de 21% à 28% massique d'alcanol comportant 14 atomes de carbone et de 4% à 8% massique d'alcanol comportant 16 atomes de carbone, préalablement chauffés, sont introduits dans un réacteur, maintenus sous agitation et séchés, On ajoute ensuite 326 grammes de trifluorure de bore à 50% dans le diéthyl-éther puis progressivement sous agitation pendant 4 heures, 32.600 g de 3-(hydroxyméthyl) 3-éthyl oxétane, en maintenant la température autour de 110°C. Le milieu réactionnel est alors laissé encore 11 heures à 115°C. On obtient alors la composition (C') attendue, caractérisée comme suit :
- Aspect à 25°C : Gel trouble
- Indice d'acide (en mg KOH/g ; NFT60-204) : 3,9
- Indice d'hydroxyle (en mg KOH/g) : 400,5
- Teneur massique en 2-(hydroxyméthyl) 2-éthyl oxétane libre (déterminée par chromatographie en phase gazeuse) : < 0.05%
- Teneur massique en alcanols libres (déterminée par chromatographie en phase gazeuse) : alcanol en C12 : 5,7% ; alcanol en C14 : 2,0% ; alcanol en C16 : 0,5%.

### Stade A2) : Préparation de la composition tensioactive (C)

50.000 g de la composition intermédiaire (C') obtenue au stade A1) précédent, sont introduits dans un autoclave d'une capacité de 0,1 m³, avec 75 g de potasse puis séchés à une température de 105°C. Une quantité de 35 000 g d'oxyde d'éthylène est ensuite progressivement introduite en régulant la température du mélange réactionnel à une valeur de 125°C. Une fois la quantité totale d'oxyde d'éthylène introduite, le mélange réactionnel est maintenu sous agitation à 125°C pendant une durée supplémentaire d'une heure. Le produit alors obtenu est ensuite refroidi à une température de 80°C et vidangé. On obtient alors la composition tensioactive (C) caractérisée comme suit :
Aspect à 30°C : liquide limpide
Couleur : 125 Alpha
Indice d'hydroxyle (en mg KOH/g) : 252,5
Indice d'acide (en mg KOH/g) (NFT60-204) : 0,08
Teneur résiduelle en eau : 0,05%
Point de trouble (NF EN 1890E) : 76°C
Teneur massique en alcanols libres (chromatographie en phase gazeuse) : Alcanol en C12 : 1,2% ; alcanol en C14 : 0,4% ; alcanol en C16 : 0,1% soit au total 1.7% d'alcanols résiduels
Viscosité à 25°C (Brookfield LVT Mobile 3 Vitesse 12) : 1.072 mPa.s

### EXEMPLE 1 (selon l'invention) : Latex inverse auto-inversible du copolymère ATBS(sel de Na) / HEA / (ALE-4 OE) [(ATBS/HEA/(ALE-4OE) 89,0/9,9/1,1 molaire] réticulé au MBA

### 1) Préparation

**a) -** Dans un premier bêcher, on introduit successivement sous agitation :
   - 672,5g d'une solution commerciale à 55% massique de sel de sodium de l'acide 2-acrylamido 2-méthyl propanesulfonique (ATBS Na),
   - 20,8g d'acrylate de 2-hydroxy éthyle (HEA) ;
   - 0,028g de méthylène bis(acrylamide) (MBA) ;et
   - 1,0 g d'une solution commerciale à 40% massique de diéthylènetriaminepenta acétate de sodium.
   Puis on y ajuste le pH à 4 en ajoutant si nécessaire la quantité requise d'acide 2-acrylamido 2-méthyl propane sulfonique et de l'eau permutée jusqu'à 700 g
b) - Dans un deuxième bêcher, on introduit successivement sous agitation : 130 g de polyisobutène,
   30 g de MARCOL^{™} 52,
   90 g d'ISOPAR^{™} H,
   17 g de MONTANE^{™} 70,
   3 g d'HYPERMER^{™} 6212,
   5 g de SIMALINE^{™}IE 200,
   7,2 g d'acrylate de lauryle tétraéthoxylé (commercial) (ALE-4OE),
   0,36g de peroxyde de dilauroyle.
c) - La phase aqueuse est alors incorporée dans la phase organique sous agitation et puis la pré-émulsion ainsi obtenue est soumise à une agitation mécanique cisaillante à l'aide d'une turbine de type Silverson de manière à créer une émulsion fine sous barbotage d'azote.
d) - Après refroidissement à environ 8 °C, la réaction de polymérisation est initiée à l'aide du couple oxydoréducteur : hydroperoxyde de cumène / métabisulfite de sodium.
e) - Une fois la réaction de polymérisation terminée, on retire par distillation sous vide l'ISOPAR^{™} H et la quasi-totalité de l'eau.
f) - On obtient après introduction de 2% massique de Laureth-7 et de 4% massique de la composition tensioactive (C) obtenue à l'exemple A, le **latex inverse auto-inversible (1)** contenant environ 63% de polymère, qui est peu visqueux, qui s'inverse très rapidement dans l'eau et dont le pouvoir épaississant est important. De plus ce latex inverse est très stable car on n'observe aucun phénomène de synérèse, en ce qu'il ne relargue que de très peu d'huile et que polymère ne sédimente pas. Sa teneur en eau mesurée par une titrimétrie Karl-Fisher est de 1,8% en poids.

### 2) Mesures de viscosité

a) - On mesure la viscosité du latex inverse auto-inversible (1) obtenu comme indiqué au paragraphe 1, celle d'une solution aqueuse exempte de chlorure de sodium (Sol.1) et celles de solutions aqueuses contenant respectivement 0,1% massique (Sol.2) et 1 % massique (Sol.3) de chlorure de sodium, lesdites solutions aqueuses comprenant chacune 2% massique dudit latex inverse auto-inversible (1). Les résultats mesurés à l'aide d'un viscosimètre Brookfield RVT sont consignés dans le tableau suivant :

| | Mobile (M) ; Vitesse de rotation du mobile (V) (en tours par minute) | Viscosité (en mPa.s) |
|---|---|---|
| Latex inverse (1) | M 3, V 20 | 2 700 |
| Sol.1 | M 6, V 5 | 54 000 |
| Sol. 2 | M 6, V 5 | 27 000 |
| Sol.3 | M 3, V 5 | 1600 |

### 3) Mesure du temps d'inversion et évaluation de la stabilité du latex inverse.

a) - Le temps d'inversion est évalué en mesurant le temps nécessaire pour obtenir un gel lisse et homogène pour une solution aqueuse à 2% massiques de latex inverse auto-inversible (1) dans les conditions standard de mesure de cette viscosité c'est-à-dire en incorporant 16 g du latex inverse (1) dans 784 g d'eau, le tout étant placé dans un bécher de 1 litre de forme basse, puis en agitant le tout à l'aide d'une hélice type papillon tournant à 150 tours par minute. Le temps d'inversion est donc la durée évaluée entre la mise en route de l'agitateur et l'apparition d'un milieu lisse et homogène dans le bêcher. Dans le présent exemple, le temps d'inversion est de 50 secondes.
b) - La stabilité du latex inverse est évaluée en observant le temps d'apparition d'une couche huile en surface. Dans le présent exemple, le temps d'apparition de la couche d'huile en surface du latex inverse (1) est de deux semaines.

### EXEMPLE T1 (selon l'état de la technique) : Latex inverse auto-inversible du copolymère ATBS(sel de Na) / HEA / (ALE-4OE) [(ATBS/HEA/(ALE-4OE) 89,0/9,9/1,1 molaire] réticulé au MBA

### 1) Préparation

On reproduit les étapes a) à d) de l'exemple 1. A l'étape f), on ajoute 4% massique de MONTANOX^{™} 20 à la place de 4% massique de la composition tensioactive (C) et l'on obtient le latex inverse auto-inversible (T1).

### 2) Mesures de viscosité

a) - On mesure la viscosité du latex inverse auto-inversible (T1) obtenu comme indiqué au paragraphe 1, celle d'une solution aqueuse exempte de chlorure de sodium (Sol.4) et celles de solutions aqueuses contenant respectivement 0,1 % massique (Sol.5) et 1 % massique (Sol.6) de chlorure de sodium, lesdites solutions aqueuses comprenant chacune 2% massique dudit latex inverse auto-inversible (T1). Les résultats mesurés à l'aide d'un viscosimètre Brookfield RVT sont consignés dans le tableau suivant :

| | Mobile (M) ; Vitesse de rotation du mobile (V) (en tours par minute) | Viscosité (en mPa.s) |
|---|---|---|
| Latex inverse (T1) | M 3, V 20 | 2.900 |
| Sol.4 | M 6, V 5 | 51.200 |
| Sol. 5 | M 6, V 5 | 25.200 |
| Sol.6 | M 3, V 5 | 1.300 |

### 3) Mesure du temps d'inversion et évaluation de la stabilité du latex inverse.

a) - Le temps d'inversion évalué de la même manière qu'à l'exemple précédent est de 2 minutes 20 secondes.
b) - La stabilité du latex inverse (T1) est évaluée de la même manière qu'à l'exemple précédent. Un relargage important d'huile est observé au bout d'une semaine.

### EXEMPLE 2 (selon l'invention) : Latex inverse auto-inversible du copolymère ATBS(sel de Na) / HEA / (ALE-4OE) [(ATBS/HEA/(ALE-4OE) 89,0/9,9/1,1 molaire] réticulé au MBA

### 1) Préparation

On reproduit les étapes a) à d) de l'exemple 1. A l'étape f), on n'ajoute que 4% massique de la composition tensioactive (C) et l'on obtient le latex inverse auto-inversible (2).

### 2) Viscosimétrie, mesure du temps d'inversion et évaluation de la stabilité du latex inverse (2)

a) - Les performances viscosimétriques du latex inverse (2) sont similaires à celles rapportées pour le latex inverse de l'exemple 1.
b) - Le temps d'inversion du latex inverse (2), évalué de la même manière qu'à l'exemple 1, est d'environ 40 secondes.
c) - La stabilité du latex inverse (2) est évaluée de la même manière qu'à l'exemple 1. Le temps d'apparition de la couche d'huile est de quelques jours.

### EXEMPLE T2 (selon l'état de la technique) : Latex inverse auto-inversible du copolymère ATBS(sel de Na) / HEA / (ALE-4OE) [(ATBS/HEA/(ALE-4OE) 89,0/9,9/1,1 molaire] réticulé au MBA

### 1) Préparation

On reproduit les étapes a) à d) de l'exemple 1. A l'étape f), on ajoute 4% massique d'une composition (C"'), qui comprend pour 100% molaire :
i) - Une proportion supérieure ou égale à 10% molaire et inférieure ou égale à 50% molaire d'un composé de formule (II'") correspondant à la formule (II) dans laquelle R2 représente un radical alkyle linéaire ou ramifié, comportant 10 atomes de carbone, et dans la quelle la somme m1 + m2 + m3 est égale à 5;
ii) - Une proportion supérieure ou égale à 50% molaire et inférieure ou égale à 90% molaire d'un composé de formule (III''') ou de son isomère de formule (IV''') ou du mélange de ces deux isomères, formules (III''') et (IV''') correspondant respectivement aux formules (III) et (IV) dans lesquelles R2 représente un radical alkyle linéaire ou ramifié, comportant 10 atomes de carbone, et dans la quelle la somme m4 + m5 + m6 + m7 est égale à 5; et l'on obtient le latex inverse auto-inversible (T2).

### 2) Viscosimétrie, mesure du temps d'inversion et évaluation de la stabilité du latex inverse (T2)

a) - Les performances viscosimétriques du latex inverse (T2) sont similaires à celles rapportées pour le latex inverse de l'exemple 1.
b) - Le temps d'inversion du latex inverse (T2), évalué de la même manière qu'à l'exemple 1, est d'environ 50 secondes.
c) - La stabilité du latex inverse (T2) est évaluée de la même manière qu'à l'exemple 1.
   Le temps d'apparition de la couche d'huile est de quelques heures.

### EXEMPLE T3 (selon l'état de la technique) : Latex inverse auto-inversible du copolymère AM / AA / (ALE-4OE) [(AM/AA/(ALE-4OE) 24,7/74,1/1,2 molaire] réticulé au MBA

### 1) Préparation

a) - Dans un premier bêcher, on introduit successivement sous agitation :
   - 106,5 g d'une solution commerciale d'acrylamide (AM) à 50% (massique)
   - 162,0 g d'acide acrylique glacial (AA),
   - 98,1 g d'une solution d'ammoniaque à 29,3% en poids,
   - 0,047 g de méthylène bis(acrylamide) (MBA).
   - 0,45 g d'une solution commerciale à 40% du diéthylènetriaminepentaacétate de sodium,
   - de l'eau permutée jusqu'à 680 g.
b) - Dans un deuxième bêcher, on introduit successivement sous agitation :
   - 121 g de polyisobutène,
   - 28 g de MARCOL^{™} 52,
   - 99 g d'ISOPAR^{™} H,
   - 17 g de MONTANE^{™} 70,
   - 3 g d'HYPERMER^{™} 2296,
   - 5 g de SIMALINE^{™} IE 200,
   - 1,2 g d'acrylate de lauryle tétraéthoxylé (commercial) (ALE-4OE),
   - 0,1 g d'AIBN.
c) - La phase aqueuse est alors incorporée dans la phase organique sous agitation et puis la pré-émulsion ainsi obtenue est soumise à une agitation mécanique cisaillante à l'aide d'une turbine de type Silverson de manière à créer une émulsion fine sous barbotage d'azote.
d) - Après refroidissement à environ 8 °C, la réaction de polymérisation est initiée à l'aide du couple oxydoréducteur : hydroperoxyde de cumène / métabisulfite de sodium.
e) - Une fois la réaction de polymérisation terminée, on retire par distillation sous vide l'ISOPAR^{™} H et la quasi-totalité de l'eau.
f) - On obtient après introduction de 4% de MONTANOX^{™} 20 et de 2% de Laureth 7, le latex inverse auto-inversible (T3), contenant environ 63% de polymère, qui est peu visqueux, qui s'inverse très rapidement dans l'eau et dont le pouvoir épaississant est important. Sa teneur en eau mesurée par une titrimétrie Karl-Fisher est de 1,8% en poids.

### 2) Mesures de viscosité

a) - On mesure la viscosité du latex inverse auto-inversible (T3) obtenu comme indiqué au paragraphe 1, celle d'une solution aqueuse exempte de chlorure de sodium (Sol.7) et celles de solutions aqueuses contenant respectivement 0,1% massique (Sol.8) et 1% massique (Sol.9) de chlorure de sodium, lesdites solutions aqueuses comprenant chacune 2% massique dudit latex inverse auto-inversible (T3). Les résultats mesurés à l'aide d'un viscosimètre Brookfield RVT sont consignés dans le tableau suivant :

| | Mobile (M) ; Vitesse de rotation du mobile (V) (en tours par minute) | Viscosité (en mPa.s) |
|---|---|---|
| Latex inverse (T3) | | nd |
| Sol.7 | M 6, V 5 | 79 400 |
| Sol. 8 | M 6, V 5 | 45 200 |
| Sol.9 | M 3, V 5 | 3 300 |

| | | |
|---|---|---|
| nd : non déterminée | | |

### 3) Mesure du temps d'inversion et évaluation de la stabilité du latex inverse (T3).

b) - Le temps d'inversion du latex inverse (T3), évalué de la même manière qu'à l'exemple 1, est d'environ 2 minutes.
c) - La stabilité du latex inverse du latex inverse (T3) est évaluée de la même manière qu'à l'exemple 1. Le temps d'apparition de la couche d'huile est de deux semaines.

### EXEMPLE 3 (selon l'invention) : Latex inverse auto-inversible du copolymère AM / AA / (ALE-4OE) [(AM/AA/(ALE-4OE) 24,7/74,1/1,2 molaire] réticulé au MBA

### 1) Préparation

On reproduit les étapes a) à d) de l'exemple T3. A l'étape f), on ajoute 2% massique de Laureth 7 et 4% massique de la composition tensioactive (C) au lieu des de 4% de MONTANOX^{™} 20 et 2% de Laureth 7 dudit exemple T3 et l'on obtient le latex inverse auto-inversible (3).

### 2) Viscosimétrie, mesure du temps d'inversion et évaluation de la stabilité du latex inverse 3)

a) - Les performances viscosimétriques du latex inverse (3) sont similaires à celles rapportées pour le latex inverse de l'exemple T3.
b) - Le temps d'inversion du latex inverse (3), évalué de la même manière qu'à l'exemple 1, est d'environ 30 secondes.
c) - La stabilité du latex inverse (3) est évaluée de la même manière qu'à l'exemple 1.
   Le temps d'apparition des premières gouttes d'huile est de trois semaines.

### EXEMPLE 4 (selon l'invention) : Latex inverse auto-inversible du copolymère ATBS (sel de Na) / AA / HEA / THAM / (ALE-4OE [ATBS/AA/HEA/THAM/(ALE-4OE) 83,9/1.9/9,3/3,7/1,2 molaire] réticulé au MBA

### 1) Préparation

a) - Dans un premier bêcher, on introduit successivement sous agitation :
   - 672,5 g d'une solution commerciale à 55% (massique) du sel de sodium de l'acide 2 acrylamido 2 méthyl propanesulfonique (ATBSNa) ;
   - 20,8 g d'acrylate de 2-hydroxy éthyle ;
   - 12,6g de THAM;
   - 2,6 g d'acide acrylique (AA)
   - 0,041 g de méthylène bis(acrylamide) (MBA).
   - 0,45 g d'une solution commerciale à 40% du diéthylènetriaminepentacétate de sodium,
   Puis on y ajuste le pH à 4 en ajoutant si nécessaire la quantité requise d"acide 2 acrylamido 2 méthyl propane sulfonique et de l'eau permutée jusqu'à 700 g
b) - Dans un deuxième bêcher, on introduit successivement sous agitation :
   - 130 g de polyisobutène,
   - 30 g de MARCOL^{™} 52,
   - 90 g d'ISOPAR^{™} H,
   - 17 g de MONTANE^{™} 70,
   - 5 g d'HYPERMER^{™} 6212,
   - 3g de DEHYMULS PGPH (Polyhydroxy stéarate de polyglycérol),
   - 7,4 g d'acrylate de lauryle tétraéthoxylé (commercial) (ALE-4OE),
   - 0,14g de dilauroyle peroxyde
c) - La phase aqueuse est alors introduite dans la phase organique sous agitation et puis la pré-émulsion ainsi obtenue est soumise à une agitation mécanique cisaillante à l'aide d'une turbine de type Silverson de manière à créer une émulsion fine sous barbotage d'azote.
d) - Après refroidissement à environ 8 °C, la réaction de polymérisation est initiée à l'aide du couple oxydoréducteur : hydroperoxyde de cumène / métabisulfite de sodium.
e) - Une fois la réaction de polymérisation terminée, on retire par distillation sous vide l'ISOPAR^{™} H et la quasi-totalité de l'eau.
f) - On obtient après introduction de 2% massique de Laureth-7 et de 4% massique de la composition tensioactive (C) obtenue à l'exemple A, le latex inverse auto-inversible (4), contenant environ 63% de polymère, qui est peu visqueux, qui s'inverse très rapidement dans l'eau et dont le pouvoir épaississant est important. Sa teneur en eau mesurée par une titrimétrie Karl-Fisher est de 2,2% en poids.

### 2) Mesures de viscosité

a) - On mesure la viscosité du latex inverse auto-inversible (4) obtenu comme indiqué au paragraphe 1, celle d'une solution aqueuse exempte de chlorure de sodium (Sol.10) et celle d'une solution aqueuse contenant 0,1% massique (Sol.11) de chlorure de sodium, lesdites solutions aqueuses comprenant chacune 2% massique dudit latex inverse auto-inversible (4). Les résultats mesurés à l'aide d'une viscosimètre Brookfield RVT sont consignés dans le tableau suivant :

| | Mobile (M) ; Vitesse de rotation du mobile (V) (en tours par minute) | Viscosité (en mPas) |
|---|---|---|
| Latex inverse (4) | M3, V20 | 1.100 |
| Sol.10 | M6, V5 | 66.200 |
| Sol.11 | M6, V5 | 16.500 |

| | | |
|---|---|---|
| nd : non déterminée | | |

### 3) Mesure du temps d'inversion et évaluation de la stabilité du latex inverse.

b) - Le temps d'inversion du latex inverse (4), évalué de la même manière qu'à l'exemple 1, est d'environ 30 secondes.
c) - La stabilité du latex inverse (4) est évaluée de la même manière qu'à l'exemple 1.
   Le temps d'apparition des premières gouttes d'huile est de trois semaines.

## Revendications

1. Composition tensioactive (C), comprenant pour 100% molaire :
1) - Une proportion supérieure ou égale à 10% molaire et inférieure ou égale à 50% molaire d'une composition (C_{II}) comprenant pour 100% molaire :
α) - De 60% molaire à 100% molaire d'un composé de formule (II) : 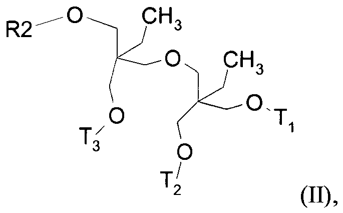 dans laquelle :
- R2 représente un radical alkyle linéaire ou ramifié, comportant de 12 atomes de carbone,
- T₁, représente un atome d'hydrogène ou un radical (-CH₂-CH₂-O-)ₘ₁-H dans lequel m1 est un nombre entier supérieur ou égal à zéro et inférieur ou égal à dix,
- T₂ identique ou différent de T₁, représente un atome d'hydrogène ou un radical (-CH₂-CH₂-O-)ₘ₂-H dans lequel m2 est un nombre entier supérieur ou égal à zéro et inférieur ou égal à dix, et
- T₃ identique ou différent de T₁ et de T₂, représente un atome d'hydrogène ou un radical (-CH₂-CH₂-O-)ₘ₃-H dans lequel m3 est un nombre entier supérieur ou égal à zéro et inférieur ou égal à dix,
- étant entendu que la somme m1 + m2 + m3 est supérieure à 0 et inférieure ou égale à dix ;
β) - Optionnellement jusqu'à 40% molaire d'un composé de formule (II') 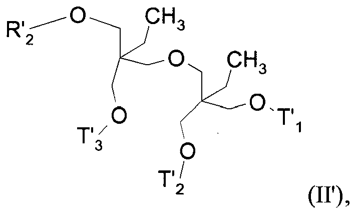 dans laquelle :
- R'₂ représente un radical alkyle linéaire ou ramifié, comportant 14 atomes de carbone,
- T'₁, représente un atome d'hydrogène ou un radical (-CH₂-CH₂-O-)ₘ₁-H dans lequel m1 est un nombre entier supérieur ou égal à zéro et inférieur ou égal à dix,
- T'₂ identique ou différent de T'₁, représente un atome d'hydrogène ou un radical (-CH₂-CH₂-O-)ₘ₂-H dans lequel m2 est un nombre entier supérieur ou égal à zéro et inférieur ou égal à dix, et
- T'₃ identique ou différent de T'₁ et de T'₂, représente un atome d'hydrogène ou un radical (-CH₂-CH₂-O-)ₘ₃-H dans lequel m3 est un nombre entier supérieur ou égal à zéro et inférieur ou égal à dix,
- étant entendu, que la somme m1 + m2 + m3 est supérieure à 0 et inférieure ou égale à dix ; et
γ) - Optionnellement jusqu'à 10% molaire d'un composé de formule (II") : 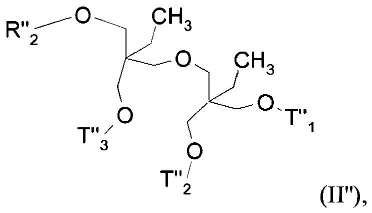 dans laquelle :
- R"₂ représente un radical alkyle linéaire ou ramifié, comportant 16 atomes de carbone,
- T"₁, représente un atome d'hydrogène ou un radical (-CH₂-CH₂-O-)ₘ₁-H dans lequel m1 est un nombre entier supérieur ou égal à zéro et inférieur ou égal à dix,
- T"₂ identique ou différent de T"₁, représente un atome d'hydrogène ou un radical (-CH₂-CH₂-O-)ₘ₂-H dans lequel m2 est un nombre entier supérieur ou égal à zéro et inférieur ou égal à dix, et
- T"₃ identique ou différent de T"₁ et de T"₂, représente un atome d'hydrogène ou un radical (-CH₂-CH₂-O-)ₘ₃-H dans lequel m3 est un nombre entier supérieur ou égal à zéro et inférieur ou égal à dix,
- étant entendu que la somme m1 + m2 + m3 est supérieure à 0 et inférieure ou égale à dix ;
2) - Une proportion supérieure ou égale à 50% molaire et inférieure ou égale à 90% molaire d'une composition (C_{III}) comprenant pour 100% molaire :
α) - De 60% molaire à 100% molaire d'un composé de formule (III) : 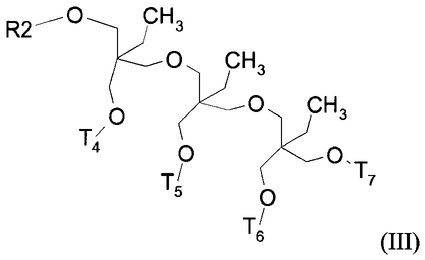 ou de son isomère de formule (IV) : 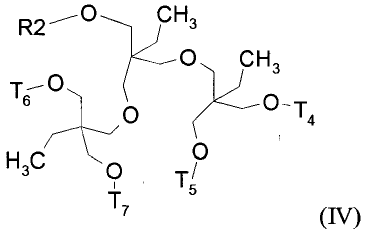 ou du mélange de ces deux isomères,
formules (III) et (IV) dans lesquelles :
- R2 représente un radical alkyle linéaire ou ramifié, comportant 12 atomes de carbone,
- T₄, représente un atome d'hydrogène ou un radical (-CH₂-CH₂-O-)ₘ₄-H dans lequel m4 est un nombre entier supérieur ou égal à zéro et inférieur ou égal à dix,
- T₅ identique ou différent de T₄, représente un atome d'hydrogène ou un radical (-CH₂-CH₂-O-)ₘ₅-H dans lequel m5 est un nombre entier supérieur ou égal à zéro et inférieur ou égal à dix,
- T₆ identique ou différent de T₄ et de T₅ représente un atome d'hydrogène ou un radical (-CH₂-CH₂-O-)ₘ₆-H dans lequel m6 est un nombre entier supérieur ou égal à zéro et inférieur ou égal à dix,
- T₇ identique ou différent de T₄ de T₅ et de T₆, représente un atome d'hydrogène ou un radical (-CH₂-CH₂-O-)ₘ₇-H dans lequel m7 est un nombre entier supérieur ou égal à zéro et inférieur ou égal à dix étant entendu que la somme m4 + m5 + m6 + m7 est supérieure à 0 et inférieure ou égale à dix ;
P) - Optionnellement jusqu'à 40% molaire composé de formule (III') : 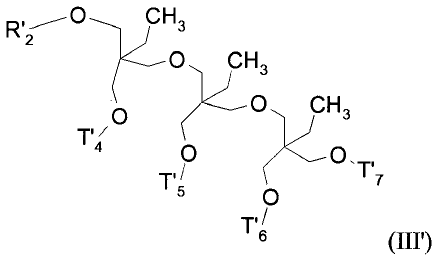 ou de son isomère de formule (IV') : 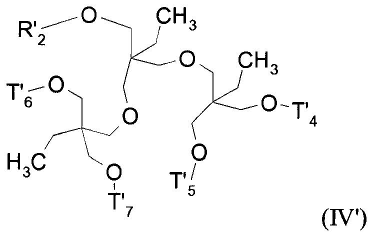 ou du mélange de ces deux isomères,
formules (III') et (IV') dans lesquelles :
- R'₂ représente un radical alkyle linéaire ou ramifié, comportant 14 atomes de carbone,
- T'₄, représente un atome d'hydrogène ou un radical (-CH₂-CH₂-O-)ₘ₄-H dans lequel m4 est un nombre entier supérieur ou égal à zéro et inférieur ou égal à dix,
- T'₅ identique ou différent de T'₄, représente un atome d'hydrogène ou un radical (-CH₂-CH₂-O-)ₘ₅-H dans lequel m5 est un nombre entier supérieur ou égal à zéro et inférieur ou égal à dix,
- T'₆ identique ou différent de T'₄ et de T'₅, représente un atome d'hydrogène ou un radical (-CH₂-CH₂-O-)ₘ₆-H dans lequel m6 est un nombre entier supérieur ou égal à zéro et inférieur ou égal à dix, et
- T'₇ identique ou différent de T'₄, de T'₅ et de T'₆, représente un atome d'hydrogène ou un radical (-CH₂-CH₂-O-)ₘ₇-H dans lequel m7 est un nombre entier supérieur ou égal à zéro et inférieur ou égal à dix,
- étant entendu que la somme m4 + m5 + m6 + m7 est supérieure à 0 et inférieure ou égale à dix ; et
γ) - Optionnellement jusqu'à 10% molaire d'un composé de formule (III") : 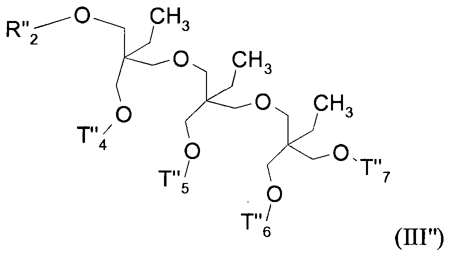 ou de son isomère de formule (IV") : 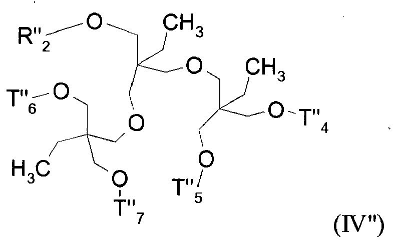 ou du mélange de ces deux isomères,
formules (III") et (IV") dans lesquelles :
- R"₂ représente un radical alkyle linéaire ou ramifié, comportant 16 atomes de carbone,
- T"₄, représente un atome d'hydrogène ou un radical (-CH₂-CH₂-O-)ₘ₄-H dans lequel m4 est un nombre entier supérieur ou égal à zéro et inférieur ou égal à dix,
- T"₅ identique ou différent de T"₄, représente un atome d'hydrogène ou un radical (-CH₂-CH₂-O-)ₘ₅-H dans lequel m5 est un nombre entier supérieur ou égal à zéro et inférieur ou égal à dix,
- T"₆ identique ou différent de T"₄ et de T"₅ représente un atome d'hydrogène ou un radical (-CH₂-CH₂-O-)ₘ₆-H dans lequel m6 est un nombre entier supérieur ou égal à zéro et inférieur ou égal à dix, et
- T"₇ identique ou différent de T"₄, de T"₅ et de T"₆, représente un atome d'hydrogène ou un radical (-CH₂-CH₂-O-)ₘ₇-H dans lequel m7 est un nombre entier supérieur ou égal à zéro et inférieur ou égal à dix,
- étant entendu que la somme m4 + m5 + m6 + m7 est supérieure à 0 et inférieure ou égale à dix.

2. Composition tensioactive (C) telle que définie à la revendication 1, comprenant en ou-tre :
3) - Jusqu'à 5% molaire d'une composition (C_{V}) comprenant pour 100% molaire
α) - De 60% molaire à 100% molaire d'un composé de formule (V) : 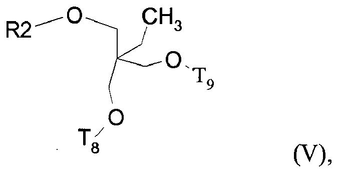 dans laquelle :
- R2 représente un radical alkyle linéaire ou ramifié, comportant de 12 atomes de carbone,
- T₈, représente un atome d'hydrogène ou un radical (-CH₂-CH₂-O-)ₘ₈-H dans lequel m8 est un nombre entier supérieur ou égal à zéro et inférieur ou égal à dix,
- T₉ identique ou différent de T_{8,} représente un atome d'hydrogène ou un radical (-CH₂-CH₂-O-)ₘ₉-H dans lequel m9 est un nombre entier supérieur ou égal à zéro et inférieur ou égal à dix, et
- étant entendu que la somme m8 + m9 est supérieure à 0 et inférieure ou égale à dix ;
β) - Optionnellement jusqu'à 40% molaire d'un composé de formule (V') 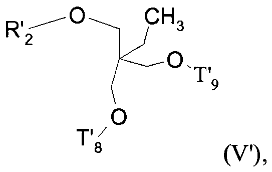 dans laquelle :
- R'₂ représente un radical alkyle linéaire ou ramifié, comportant 14 atomes de carbone,
- T'₈, représente un atome d'hydrogène ou un radical (-CH₂-CH₂-O-)ₘ₈-H dans lequel m8 est un nombre entier supérieur ou égal à zéro et inférieur ou égal à dix,
- T'₉ identique ou différent de T'₈, représente un atome d'hydrogène ou un radical (-CH₂-CH₂-O-)ₘ₉-H dans lequel m9 est un nombre entier supérieur ou égal à zéro et inférieur ou égal à dix, et
- étant entendu que la somme m8 + m9 est supérieure à 0 et inférieure ou égale à dix ; et
γ) - Optionnellement jusqu'à 10% molaire d'un composé de formule (V") : 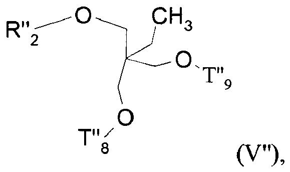 dans laquelle :
- R"₂ représente un radical alkyle linéaire ou ramifié, comportant 16 atomes de carbone,
- T"₈, représente un atome d'hydrogène ou un radical (-CH₂-CH₂-O-)ₘ₈-H dans lequel m8 est un nombre entier supérieur ou égal à zéro et inférieur ou égal à dix,
- T"₉ identique ou différent de T"₈, représente un atome d'hydrogène ou un radical (-CH₂-CH₂-O-)ₘ₉-H dans lequel m9 est un nombre entier supérieur ou égal à zéro et inférieur ou égal à dix, et
- étant entendu que la somme m8 + m9 est supérieure à 0 et inférieure ou égale à dix.

3. Composition active (C) telle que définie à l'une quelconque des revendications 1 ou 2, comprenant en outre :
4) - Jusqu'à 5% molaire d'une composition (C_{VI}) comprenant pour 100% molaire :
α) - De 60% molaire à 100% molaire d'un composé de formule (VI) :
R2-(-CH₂-CH₂-O-)ₘ₁₀-H (VI),
dans laquelle R2 représente un radical alkyle linéaire ou ramifié, comportant 12 atomes de carbone et m10 est un nombre entier supérieur ou égal à zéro et inférieur ou égal à dix ;
β) - Optionnellement jusqu'à 40% molaire d'un composé de formule (VI') :
R'₂-(-CH₂-CH₂-O-)ₘ₁₀-H (VI'),
dans laquelle R'₂ représente un radical alkyle linéaire ou ramifié, comportant 14 atomes de carbone et m10 est un nombre entier supérieur ou égal à zéro et inférieur ou égal à dix ; et
γ) - Optionnellement jusqu'à 10% molaire d'un composé de formule (VI") :
R"₂-(-CH₂-CH₂-O-)ₘ₁₀-H (VI"),
dans laquelle R"₂ représente un radical alkyle linéaire ou ramifié, comportant 16 atomes de carbone et m10 est un nombre entier supérieur ou égal à zéro et inférieur ou égal à dix.

4. Composition tensioactive (C) telle que définie à l'une quelconque des revendications 1 à 3, **caractérisée en ce qu'**elle comprend :
1) - Une proportion supérieure ou égale à 20% molaire et inférieure ou égale à 50% molaire d'une composition (C_{II}) telle que définie précédemment ;
2) - Une proportion supérieure ou égale à 50% molaire et inférieure ou égale à 80% molaire d'une composition (C_{III}) telle que définie précédemment.

5. Composition tensioactive (C) telle que définie à l'une quelconque des revendications 1 à 4, **caractérisée en ce que** :
1) - ladite composition (C_{II}) comprend pour 100% molaire :
α) - De 60% à 80% molaire du composé de formule (II),
β) - De 15% à 30% molaire du composé de formule (II'), et
γ) - Jusqu'à 10% molaire du composé de formule (II"), et
2) - ladite composition (C_{III}) comprend pour 100% molaire :
α) - De 60% à 80% molaire du composé de formule (III), de son isomère de formule (IV) ou du mélange de ces isomères,
β) - de 15% à 30% molaire du composé de formule (III'), de son isomère de formule (IV') ou du mélange de ces isomères, et
γ) - Jusqu'à 10% molaire du composé de formule (III"), de son isomère de formule (IV") ou du mélange de ces isomères.

6. Procédé de préparation de la composition tensioactive (C) telle que définie à l'une quelconque des revendications 1 à 5, **caractérisé en ce qu'**il comprend les étapes successive suivantes :
**Une étape a)** de réaction d'un mélange d'alcools comprenant pour 100% molaire :
- De 60% molaire à 100% molaire d'un composé de formule (VII),
R2-O-H (VII),
dans laquelle R2 représente un radical alkyle linéaire ou ramifié, comportant 12 atomes de carbone ;
- Optionnellement jusqu'à 40% molaire d'un composé de formule (VII') :
R'₂-O-H (VII'),
dans laquelle R'₂ représente un radical alkyle linéaire ou ramifié, comportant 14 atomes de carbone, et
- Optionnellement jusqu'à 10% molaire d'un composé de formule (VII") :
R"₂-O-H (VII"),
dans laquelle R"₂ représente un radical alkyle linéaire ou ramifié, comportant 16 atomes de carbone;
avec un excès de 3-(hydroxyméthyl) 3-éthyl oxétane de formule (VIII) : 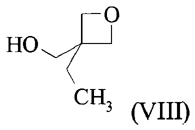 pour former une composition (C') comprenant :
α) - Le composé de formule (IX) : 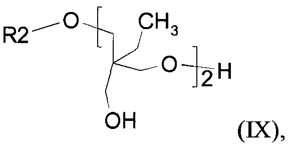 dans laquelle R2 est tel que défini ci-dessus ;
β) - Optionnellement le composé de formule (IX)' : 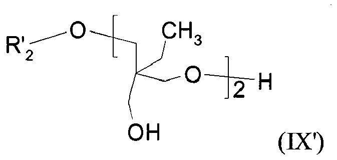 dans laquelle R'₂ est tel que défini ci-dessus ;
γ) - Optionnellement le composé de formule (IX") : 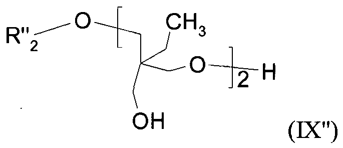 dans laquelle R"₂ est tel que défini ci-dessus ;
δ) - Le composé de formule (X) : 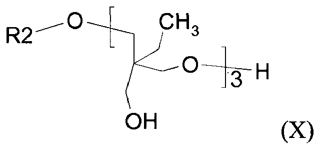 ou son isomère de formule (XI) : 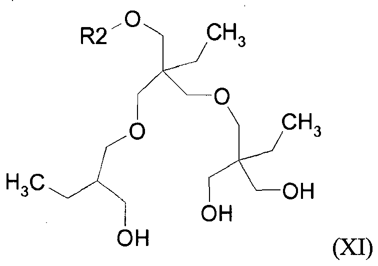 ou un mélange de ces deux isomères ; composés de formules (X) et (XI) dans lesquelles R2 est tel que défini ci-dessus ;
ε) - Optionnellement le composé de formule (X') : 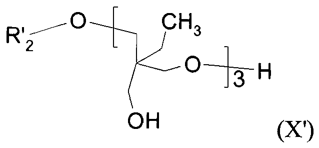 ou son isomère de formule (XI') : 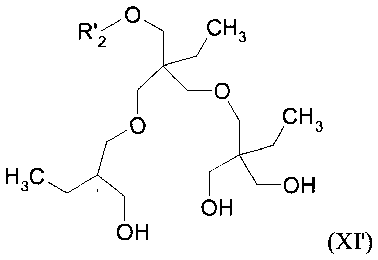 ou un mélange de ces deux isomères ; composés de formules (X') et (XI') dans lesquelles R'₂ est tel que défini ci-dessus ;
ζ) - Optionnellement le composé de formule (X") : 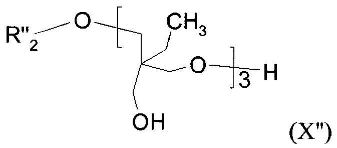 ou son isomère de formule (XI") : 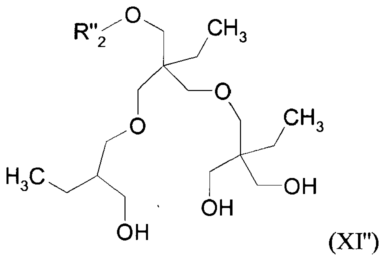 ou un mélange de ces deux isomères ; composés de formules (X") et (XI") dans lesquelles R"₂ est tel que défini ci-dessus ;
**Une étape b)** de réaction de ladite composition (C') avec l'oxyde d'éthylène de formule (XII) : 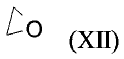 pour former ladite composition tensioactive (C) telle que définie précédemment.

7. composition (C'), intermédiaire du procédé tel que défini à la revendication 6.

8. Composés de formules (IX), (X) et (XI), intermédiaires du procédé tel que défini à la revendication 6.

9. Utilisation de la composition tensioactive (C), telle que définie à l'une quelconque des revendications 1 à 5, comme agent émulsionnant de type huile dans eau (H/E).

10. Utilisation telle que définie à la revendication 9, de la composition tensioactive (C), comme agent inverseur d'un latex inverse auto-inversible, seul ou mélange avec d'autres agent émulsionnants aptes et destinés à préparer un système émulsionnant (S₂) de type huile dans eau (H/E).

## Patentansprüche

1. Tensidzusammensetzung (C), enthaltend für 100 Mol-%:
1) einen Anteil von größer oder gleich 10 Mol-% und kleiner oder gleich 50 Mol-% einer Zusammensetzung (C_{II}), enthaltend für 100 Mol-%:
α) 60 Mol-% bis 100 Mol-% einer Verbindung der Formel (II): 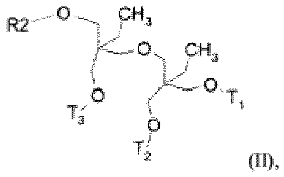 worin:
- R2 ein geradkettiges oder verzweigtes Alkylradikal mit 12 Kohlenstoffatomen darstellt,
- T₁ ein Wasserstoffatom oder ein (-CH₂-CH₂-O-)ₘ₁-H-Radikal darstellt, wobei m1 eine ganze Zahl ist, die größer oder gleich null und kleiner oder gleich zehn ist,
- T₂, das mit T₁ identisch oder von ihm verschieden ist, ein Wasserstoffatom oder ein (-CH₂-CH₂-O-)ₘ₂-H-Radikal darstellt, wobei m2 eine ganze Zahl ist, die größer oder gleich null und kleiner oder gleich zehn ist, und
- T₃, das mit T₁ und T₂ identisch oder von ihnen verschieden ist, ein Wasserstoffatom oder ein (-CH₂-CH₂-O-)ₘ₃-H-Radikal darstellt, wobei m3 eine ganze Zahl ist, die größer oder gleich null und kleiner oder gleich zehn ist,
- wobei die Summe aus m1 + m2 + m3 größer als 0 und kleiner oder gleich zehn ist;
β) optional bis zu 40 Mol-% einer Verbindung der Formel (II'): 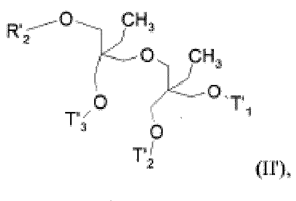 worin:
- R'₂ ein geradkettiges oder verzweigtes Alkylradikal mit 14 Kohlenstoffatomen darstellt,
- T'₁ ein Wasserstoffatom oder ein (-CH₂-CH₂-O-)ₘ₁-H-Radikal darstellt, wobei m1 eine ganze Zahl ist, die größer oder gleich null und kleiner oder gleich zehn ist,
- T'₂, das mit T'₁ identisch oder von ihm verschieden ist, ein Wasserstoffatom oder ein (-CH₂-CH₂-O-)ₘ₂-H-Radikal darstellt, wobei m2 eine ganze Zahl ist, die größer oder gleich null und kleiner oder gleich zehn ist, und
- T'₃, das mit T'₁ und T'₂ identisch oder von ihnen verschieden ist, ein Wasserstoffatom oder ein (-CH₂-CH₂-O-)ₘ₃-H-Radikal darstellt, wobei m3 eine ganze Zahl ist, die größer oder gleich null und kleiner oder gleich zehn ist,
- wobei die Summe aus m1 + m2 + m3 größer als 0 und kleiner oder gleich zehn ist; und
γ) optional bis zu 10 Mol-% einer Verbindung der Formel (II"): 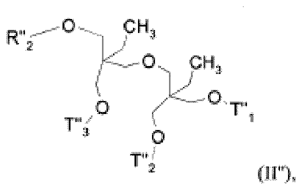 worin:
- R"₂ ein geradkettiges oder verzweigtes Alkylradikal mit 16 Kohlenstoffatomen darstellt,
- T"₁ ein Wasserstoffatom oder ein (-CH₂-CH₂-O-)ₘ₁-H-Radikal darstellt, wobei m1 eine ganze Zahl ist, die größer oder gleich null und kleiner oder gleich zehn ist,
- T"₂, das mit T"₁ identisch oder von ihm verschieden ist, ein Wasserstoffatom oder ein (-CH₂-CH₂-O-)ₘ₂-H-Radikal darstellt, wobei m2 eine ganze Zahl ist, die größer oder gleich null und kleiner oder gleich zehn ist, und
- T"₃, das mit T"₁ und T"₂ identisch oder von ihnen verschieden ist, ein Wasserstoffatom oder ein (-CH₂-CH₂-O-)ₘ₃-H-Radikal darstellt, wobei m3 eine ganze Zahl ist, die größer oder gleich null und kleiner oder gleich zehn ist,
- wobei die Summe aus m1 + m2 + m3 größer als 0 und kleiner oder gleich zehn ist;
2) einen Anteil von größer oder gleich 50 Mol-% und kleiner oder gleich 90 Mol-% einer Zusammensetzung (C_{III}), enthaltend für 100 Mol-%:
α) 60 Mol-% bis 100 Mol-% einer Verbindung der Formel (III): 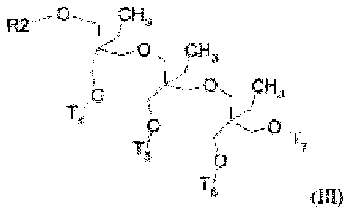 oder deren Isomers der Formel (IV): 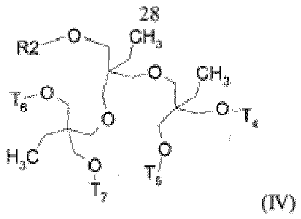 oder des Gemischs dieser zwei Isomere, wobei in den Formeln (III) und (IV):
- R2 ein geradkettiges oder verzweigtes Alkylradikal mit 12 Kohlenstoffatomen darstellt,
- T₄ ein Wasserstoffatom oder ein (-CH₂-CH₂-O-)ₘ₄-H-Radikal darstellt, wobei m4 eine ganze Zahl ist, die größer oder gleich null und kleiner oder gleich zehn ist,
- T₅, das mit T₄ identisch oder von ihm verschieden ist, ein Wasserstoffatom oder ein (-CH₂-CH₂-O-)ₘ₅-H-Radikal darstellt, wobei m5 eine ganze Zahl ist, die größer oder gleich null und kleiner oder gleich zehn ist,
- T₆, das mit T₄ und T₅ identisch oder von ihnen verschieden ist, ein Wasserstoffatom oder ein (-CH₂-CH₂-O-)ₘ₆-H-Radikal darstellt, wobei m6 eine ganze Zahl ist, die größer oder gleich null und kleiner oder gleich zehn ist,
- T₇, das mit T₄, T₅ und T₆ identisch oder von ihnen verschieden ist, ein Wasserstoffatom oder ein (-CH₂-CH₂-O-)ₘ₇-H-Radikal darstellt, wobei m7 eine ganze Zahl ist, die größer oder gleich null und kleiner oder gleich zehn ist, wobei die Summe aus m4 + m5 + m6 + m7 größer als 0 und kleiner oder gleich zehn ist;
β) optional bis zu 40 Mol-% einer Verbindung der Formel (III'): 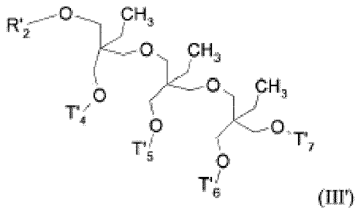 oder deren Isomers der Formel (IV'): 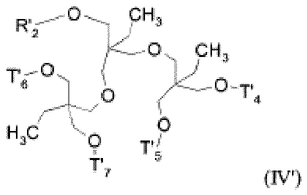 oder des Gemischs dieser zwei Isomere, wobei in den Formeln (III') und (IV'):
- R'₂ ein geradkettiges oder verzweigtes Alkylradikal mit 14 Kohlenstoffatomen darstellt,
- T'₄ ein Wasserstoffatom oder ein (-CH₂-CH₂-O-)ₘ₄-H-Radikal darstellt, wobei m4 eine ganze Zahl ist, die größer oder gleich null und kleiner oder gleich zehn ist,
- T'₅, das mit T'₄ identisch oder von ihm verschieden ist, ein Wasserstoffatom oder ein (-CH₂-CH₂-O-)ₘ₅-H-Radikal darstellt, wobei m5 eine ganze Zahl ist, die größer oder gleich null und kleiner oder gleich zehn ist,
- T'₆, das mit T'₄ und T'₅ identisch oder von ihnen verschieden ist, ein Wasserstoffatom oder ein (-CH₂-CH₂-O-)ₘ₆-H-Radikal darstellt, wobei m6 eine ganze Zahl ist, die größer oder gleich null und kleiner oder gleich zehn ist, und
- T'₇, das mit T'₄, T'₅ und T'₆ identisch oder von ihnen verschieden ist, ein Wasserstoffatom oder ein (-CH₂-CH₂-O-)ₘ₇-H-Radikal darstellt, wobei m7 eine ganze Zahl ist, die größer oder gleich null und kleiner oder gleich zehn ist,
- wobei die Summe aus m4 + m5 + m6 + m7 größer als 0 und kleiner oder gleich zehn ist; und
γ) optional bis zu 10 Mol-% einer Verbindung der Formel (III"): 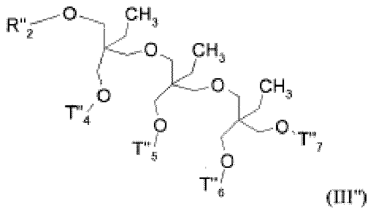 oder deren Isomers der Formel (IV"): 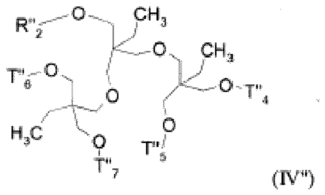 oder des Gemischs dieser zwei Isomere, wobei in den Formeln (III") und (IV"):
- R"₂ ein geradkettiges oder verzweigtes Alkylradikal mit 16 Kohlenstoffatomen darstellt,
- T"₄ ein Wasserstoffatom oder ein (-CH₂-CH₂-O-)ₘ₄-H-Radikal darstellt, wobei m4 eine ganze Zahl ist, die größer oder gleich null und kleiner oder gleich zehn ist,
- T"₅, das mit T"₄ identisch oder von ihm verschieden ist, ein Wasserstoffatom oder ein (-CH₂-CH₂-O-)ₘ₅-H-Radikal darstellt, wobei m5 eine ganze Zahl ist, die größer oder gleich null und kleiner oder gleich zehn ist,
- T"₆, das mit T"₄ und T"₅ identisch oder von ihnen verschieden ist, ein Wasserstoffatom oder ein (-CH₂-CH₂-O-)ₘ₆-H-Radikal darstellt, wobei m6 eine ganze Zahl ist, die größer oder gleich null und kleiner oder gleich zehn ist, und
- T"₇, das mit T"₄, T"₅ und T"₆ identisch oder von ihnen verschieden ist, ein Wasserstoffatom oder ein (-CH₂-CH₂-O-)ₘ₇-H-Radikal darstellt, wobei m7 eine ganze Zahl ist, die größer oder gleich null und kleiner oder gleich zehn ist,
- wobei die Summe aus m4 + m5 + m6 + m7 größer als 0 und kleiner oder gleich zehn ist.

2. Tensidzusammensetzung (C), wie in Anspruch 1 definiert, ferner umfassend:
3) bis zu 5 Mol-% einer Zusammensetzung (C_{V}), enthaltend für 100 Mol-%:
α) 60 Mol-% bis 100 Mol-% einer Verbindung der Formel (V): 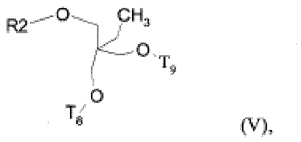 worin:
- R2 ein geradkettiges oder verzweigtes Alkylradikal mit 12 Kohlenstoffatomen darstellt,
- T₈ ein Wasserstoffatom oder ein (-CH₂-CH₂-O-)ₘ₈-H-Radikal darstellt, wobei m8 eine ganze Zahl ist, die größer oder gleich null und kleiner oder gleich zehn ist,
- T₉, das mit T₈ identisch oder von ihm verschieden ist, ein Wasserstoffatom oder ein (-CH₂-CH₂-O-)ₘ₉-H-Radikal darstellt, wobei m9 eine ganze Zahl ist, die größer oder gleich null und kleiner oder gleich zehn ist, und
- wobei die Summe aus m8 + m9 größer als 0 und kleiner oder gleich zehn ist;
β) optional bis zu 40 Mol-% einer Verbindung der Formel (V'): 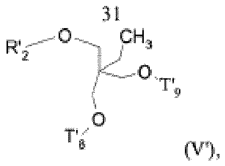 worin:
- R'₂ ein geradkettiges oder verzweigtes Alkylradikal mit 14 Kohlenstoffatomen darstellt,
- T'₈ ein Wasserstoffatom oder ein (-CH₂-CH₂-O-)ₘ₈-H-Radikal darstellt, wobei m8 eine ganze Zahl ist, die größer oder gleich null und kleiner oder gleich zehn ist,
- T'₉, das mit T'₈ identisch oder von ihm verschieden ist, ein Wasserstoffatom oder ein (-CH₂-CH₂-O-)ₘ₉-H-Radikal darstellt, wobei m9 eine ganze Zahl ist, die größer oder gleich null und kleiner oder gleich zehn ist, und
- wobei die Summe aus m8 + m9 größer als 0 und kleiner oder gleich zehn ist; und
γ) optional bis zu 10 Mol-% einer Verbindung der Formel (V"): 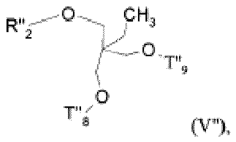 worin:
- R"₂ ein geradkettiges oder verzweigtes Alkylradikal mit 16 Kohlenstoffatomen darstellt,
- T"₈ ein Wasserstoffatom oder ein (-CH₂-CH₂-O-)ₘ₈-H-Radikal darstellt, wobei m8 eine ganze Zahl ist, die größer oder gleich null und kleiner oder gleich zehn ist,
- T"₉, das mit T"₈ identisch oder von ihm verschieden ist, ein Wasserstoffatom oder ein (-CH₂-CH₂-O-)ₘ₉-H-Radikal darstellt, wobei m9 eine ganze Zahl ist, die größer oder gleich null und kleiner oder gleich zehn ist, und
- wobei die Summe aus m8 + m9 größer als 0 und kleiner oder gleich zehn ist.

3. Aktive Zusammensetzung (C), wie in einem der Ansprüche 1 oder 2 definiert, ferner enthaltend:
4) bis zu 5 Mol-% einer Zusammensetzung (C_{VI}), enthaltend für 100 Mol-%:
α) 60 Mol-% bis 100 Mol-% einer Verbindung der Formel (VI):
R2-(-CH₂-CH₂-O-)ₘ₁₀-H (VI),
worin R2 ein geradkettiges oder verzweigtes Alkylradikal mit 12 Kohlenstoffatomen darstellt und m10 eine ganze Zahl ist, die größer oder gleich null und kleiner oder gleich zehn ist;
β) optional bis zu 40 Mol-% einer Verbindung der Formel (VI'):
R'₂-(-CH₂-CH₂-O-)ₘ₁₀-H (VI'),
worin R'₂ ein geradkettiges oder verzweigtes Alkylradikal mit 14 Kohlenstoffatomen darstellt und m10 eine ganze Zahl ist, die größer oder gleich null und kleiner oder gleich zehn ist; und
γ) optional bis zu 10 Mol-% einer Verbindung der Formel (VI"):
R"₂-(-CH₂-CH₂-O-)ₘ₁₀-H (VI"),
worin R"₂ ein geradkettiges oder verzweigtes Alkylradikal mit 16 Kohlenstoffatomen darstellt und m10 eine ganze Zahl ist, die größer oder gleich null und kleiner oder gleich zehn ist.

4. Tensidzusammensetzung (C), wie in einem der Ansprüche 1 bis 3 definiert, **dadurch gekennzeichnet, dass** sie Folgendes enthält:
1) einen Anteil von größer oder gleich 20 Mol-% und kleiner oder gleich 50 Mol-% einer Zusammensetzung (C_{II}), wie oben definiert;
2) einen Anteil von größer oder gleich 50 Mol-% und kleiner oder gleich 80 Mol-% einer Zusammensetzung (C_{III}), wie oben definiert.

5. Tensidzusammensetzung (C), wie in einem der Ansprüche 1 bis 4 definiert, **dadurch gekennzeichnet, dass**
1) die Zusammensetzung (C_{II}) für 100 Mol-% Folgendes enthält:
α) 60 Mol-% bis 80 Mol-% der Verbindung der Formel (II),
ß) 15 Mol-% bis 30 Mol-% der Verbindung der Formel (II'), und
γ) bis zu 10 Mol-% der Verbindung der Formel (II"), und
2) die Zusammensetzung (C_{III}) für 100 Mol-% Folgendes enthält:
α) 60 Mol-% bis 80 Mol-% der Verbindung der Formel (III), deren Isomers der Formel (IV) oder des Gemischs dieser Isomere,
ß) 15 Mol-% bis 30 Mol-% der Verbindung der Formel (III'), deren Isomers der Formel (IV') oder des Gemischs dieser Isomere, und
γ) bis zu 10 Mol-% der Verbindung der Formel (III"), deren Isomers der Formel (IV") oder des Gemischs dieser Isomere.

6. Verfahren zum Zubereiten der Tensidzusammensetzung (C), wie in einem der Ansprüche 1 bis 5 definiert, **dadurch gekennzeichnet, dass** es die folgenden Schritte umfasst:
einen Schritt a) der Reaktion eines Alkoholgemischs, enthaltend für 100 Mol-%:
- 60 Mol-% bis 100 Mol-% einer Verbindung der Formel (VII),
R2-O-H (VII),
worin R2 ein geradkettiges oder verzweigtes Alkylradikal mit 12 Kohlenstoffatomen darstellt;
- optional bis zu 40 Mol-% einer Verbindung der Formel (VII'):
R'₂-O-H (VII'),
worin R'₂ ein geradkettiges oder verzweigtes Alkylradikal mit 14 Kohlenstoffatomen darstellt, und
- optional bis zu 10 Mol-% einer Verbindung der Formel (VII"):
R"₂-O-H (VII"),
worin R"₂ ein geradkettiges oder verzweigtes Alkylradikal mit 16 Kohlenstoffatomen darstellt;
mit einem Überschuss von 3-(Hydroxymethyl)-3-Ethyloxetan der Formel (VIII): 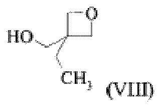 zur Bildung einer Zusammensetzung (C') enthaltend:
α) die Verbindung der Formel (IX): 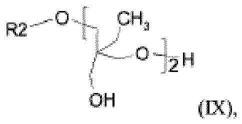 worin R2 wie oben definiert ist;
β) optional die Verbindung der Formel (IX'): 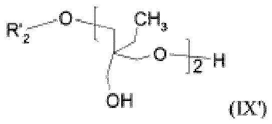 worin R'₂ wie oben definiert ist;
γ) optional die Verbindung der Formel (IX"): 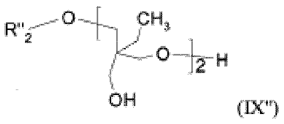 worin R"₂ wie oben definiert ist;
δ) die Verbindung der Formel (X): 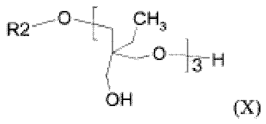 oder deren Isomers der Formel (XI): 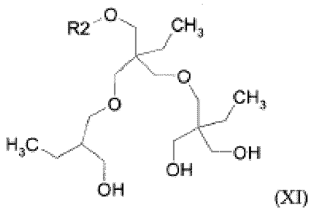 oder ein Gemisch dieser zwei Isomere; wobei in den Verbindungen der Formeln (X) und (XI) R2 wie oben definiert ist;
ε) optional die Verbindung der Formel (X'): 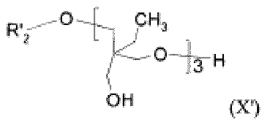 oder deren Isomers der Formel (XI'): 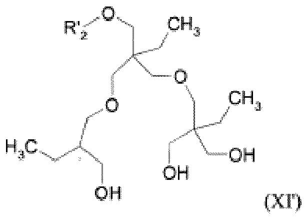 oder ein Gemisch dieser zwei Isomere; wobei in den Verbindungen der Formeln (X') und (XI') R'₂ wie oben definiert ist;
ζ) optional die Verbindung der Formel (X"): 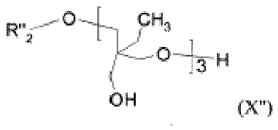 oder deren Isomers der Formel (XI"): 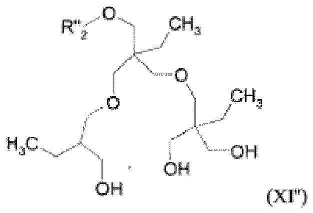 oder ein Gemisch dieser zwei Isomere; wobei in den Verbindungen der Formeln (X") und (XI") R"₂ wie oben definiert ist;
einen Schritt b) der Reaktion der Zusammensetzung (C') mit Ethylenoxid der Formel (XII): 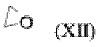 zur Bildung der Tensidzusammensetzung (C) wie oben definiert.

7. Zusammensetzung (C') als Zwischenprodukt des Verfahrens wie in Anspruch 6 definiert.

8. Verbindungen der Formeln (IX), (X) und (XI) als Zwischenprodukte des Verfahrens wie in Anspruch 6 definiert.

9. Verwendung der Tensidzusammensetzung (C), wie in einem der Ansprüche 1 bis 5 definiert, als Emulgator des Typs Öl-in-Wasser (O/W).

10. Verwendung, wie in Anspruch 9 definiert, der Tensidzusammensetzung (C) als Invertor eines inversen, auto-inversiblen Latex, allein oder in Gemisch mit anderen Emulgatoren, die für die Zubereitung eines Emulgatorsystems (S2) des Typs Öl-in-Wasser (O/W) geeignet und bestimmt sind.

## Claims

1. Surfactant composition (C) comprising, per 100 mol %:
1) a proportion of greater than or equal to 10 mol % and of less than or equal to 50 mol % of a composition (C_{II}) comprising, per 100 mol %:
α) from 60 mol % to 100 mol % of a compound of formula (II): 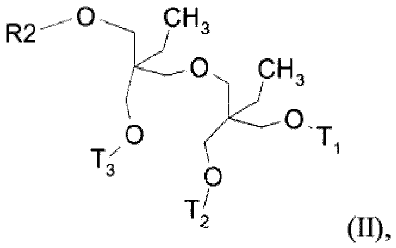 in which:
- R2 represents a linear or branched alkyl radical comprising 12 carbon atoms,
- T₁ represents a hydrogen atom or a (-CH₂-CH₂-O-)ₘ₁-H radical in which m1 is an integer of greater than or equal to zero and less than or equal to ten,
- T₂, which is identical to or different from T₁, represents a hydrogen atom or a (-CH₂-CH₂-O-)ₘ₂-H radical in which m2 is an integer of greater than or equal to zero and less than or equal to ten, and
- T₃, which is identical to or different from T₁ and T₂, represents a hydrogen atom or a (-CH₂-CH₂-O-)ₘ₃-H radical in which m3 is an integer of greater than or equal to zero and less than or equal to ten,
- it being understood that the sum m1 + m2 + m3 is greater than 0 and less than or equal to ten;
β) optionally up to 40 mol % of a compound of formula (II'): 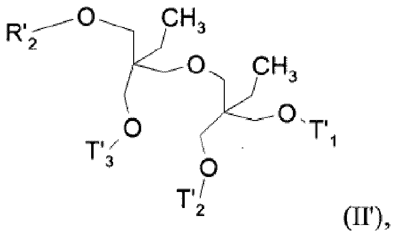 in which:
- R'₂ represents a linear or branched alkyl radical comprising 14 carbon atoms,
- T'₁ represents a hydrogen atom or a (-CH₂-CH₂-O-)ₘ₁-H radical in which m1 is an integer of greater than or equal to zero and less than or equal to ten,
- T'₂, which is identical to or different from T'₁, represents a hydrogen atom or a (-CH₂-CH₂-O-)ₘ₂-H radical in which m2 is an integer of greater than or equal to zero and less than or equal to ten, and
- T'₃, which is identical to or different from T'₁ and T'₂, represents a hydrogen atom or a (-CH₂-CH₂-O-)ₘ₃-H radical in which m3 is an integer of greater than or equal to zero and less than or equal to ten,
- it being understood that the sum m1 + m2 + m3 is greater than 0 and less than or equal to ten; and
γ) optionally up to 10 mol % of a compound of formula (II"): 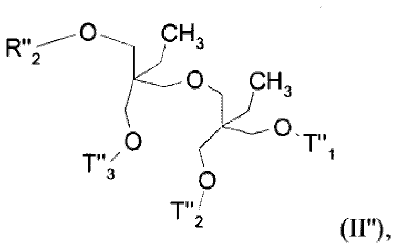 in which:
- R"₂ represents a linear or branched alkyl radical comprising 16 carbon atoms,
- T"₁ represents a hydrogen atom or a (-CH₂-CH₂-O-)ₘ₁-H radical in which m1 is an integer of greater than or equal to zero and less than or equal to ten,
- T"₂, which is identical to or different from T"₁, represents a hydrogen atom or a (-CH₂-CH₂-O-)ₘ₂-H radical in which m2 is an integer of greater than or equal to zero and less than or equal to ten, and
- T"₃, which is identical to or different from T"₁ and T"₂, represents a hydrogen atom or a (-CH₂-CH₂-O-)ₘ₃-H radical in which m3 is an integer of greater than or equal to zero and less than or equal to ten,
- it being understood that the sum m1 + m2 + m3 is greater than 0 and less than or equal to ten;
2) a proportion of greater than or equal to 50 mol % and of less than or equal to 90 mol % of a composition (C_{III}) comprising, per 100 mol %:
α) from 60 mol % to 100 mol % of a compound of formula (III): 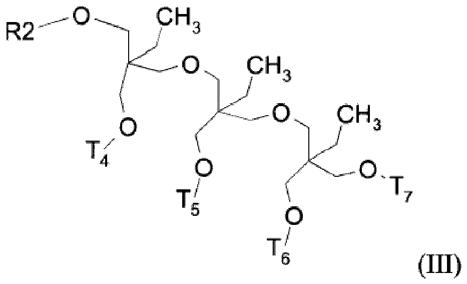 or of its isomer of formula (IV): 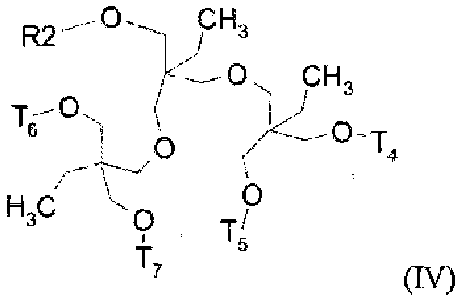 or of the mixture of these two isomers,
in which formulae (III) and (IV):
- R2 represents a linear or branched alkyl radical comprising 12 carbon atoms,
- T₄ represents a hydrogen atom or a (-CH₂-CH₂-O-)ₘ₄-H radical in which m4 is an integer of greater than or equal to zero and less than or equal to ten,
- T₅, which is identical to or different from T₄, represents a hydrogen atom or a (-CH₂-CH₂-O-)ₘ₅-H radical in which m5 is an integer of greater than or equal to zero and less than or equal to ten,
- T₆, which is identical to or different from T₄ and T₅, represents a hydrogen atom or a (-CH₂-CH₂-O-)ₘ₆-H radical in which m6 is an integer of greater than or equal to zero and less than or equal to ten,
- T₇, which is identical to or different from T₄, T₅ and T₆, represents a hydrogen atom or a (-CH₂-CH₂-O-)ₘ₇-H radical in which m7 is an integer of greater than or equal to zero and less than or equal to ten,
- it being understood that the sum m4 + m5 + m6 + m7 is greater than 0 and less than or equal to ten;
β) optionally up to 40 mol % of a compound of formula (III'): 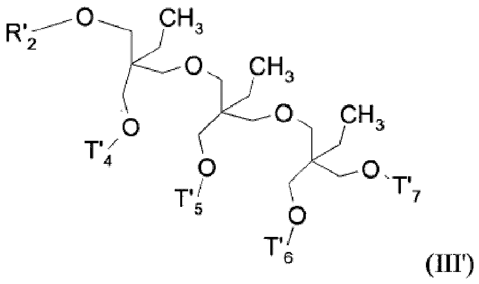 or of its isomer of formula (IV'): 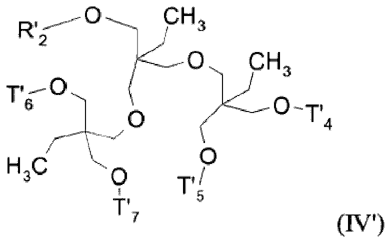 or of the mixture of these two isomers,
in which formulae (III') and (IV'):
- R'₂ represents a linear or branched alkyl radical comprising 14 carbon atoms,
- T'₄ represents a hydrogen atom or a (-CH₂-CH₂-O-)ₘ₄-H radical in which m4 is an integer of greater than or equal to zero and less than or equal to ten,
- T'₅, which is identical to or different from T'₄, represents a hydrogen atom or a (-CH₂-CH₂-O-)ₘ₅-H radical in which m5 is an integer of greater than or equal to zero and less than or equal to ten,
- T'₆, which is identical to or different from T'₄ and T'₅, represents a hydrogen atom or a (-CH₂-CH₂-O-)ₘ₆-H radical in which m6 is an integer of greater than or equal to zero and less than or equal to ten, and
- T'₇, which is identical to or different from T'₄, T'₅ and T'₆, represents a hydrogen atom or a (-CH₂-CH₂-O-)ₘ₇-H radical in which m7 is an integer of greater than or equal to zero and less than or equal to ten,
- it being understood that the sum m4 + m5 + m6 + m7 is greater than 0 and less than or equal to ten; and
γ) optionally up to 10 mol % of a compound of formula (III"): 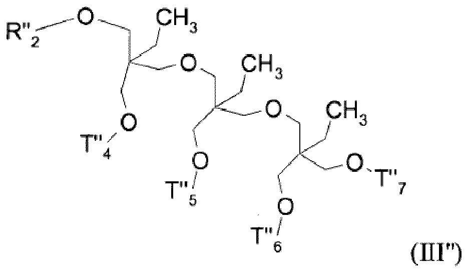 or of its isomer of formula (IV"): 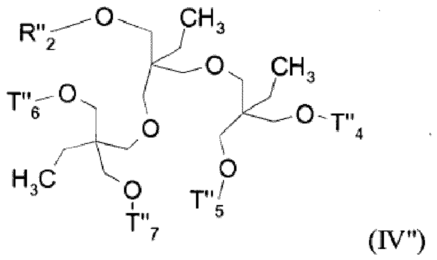 or of the mixture of these two isomers,
in which formulae (III") and (IV"):
- R"₂ represents a linear or branched alkyl radical comprising 16 carbon atoms,
- T"₄ represents a hydrogen atom or a (-CH₂-CH₂-O-)ₘ₄-H radical in which m4 is an integer of greater than or equal to zero and less than or equal to ten,
- T"₅, which is identical to or different from T"₄, represents a hydrogen atom or a (-CH₂-CH₂-O-)ₘ₅-H radical in which m5 is an integer of greater than or equal to zero and less than or equal to ten,
- T"₆, which is identical to or different from T"₄ and T"₅, represents a hydrogen atom or a (-CH₂-CH₂-O-)ₘ₆-H radical in which m6 is an integer of greater than or equal to zero and less than or equal to ten, and
- T"₇, which is identical to or different from T"₄, T"₅ and T"₆, represents a hydrogen atom or a (-CH₂-CH₂-O-)ₘ₇-H radical in which m7 is an integer of greater than or equal to zero and less than or equal to ten,
- it being understood that the sum m4 + m5 + m6 + m7 is greater than 0 and less than or equal to ten.

2. Surfactant composition (C) as defined in claim 1, additionally comprising:
3) up to 5 mol % of a composition (C_{V}) comprising, per 100 mol %:
α) from 60 mol % to 100 mol % of a compound of formula (V): 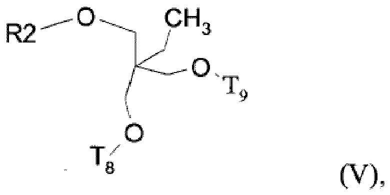 in which:
- R2 represents a linear or branched alkyl radical comprising 12 carbon atoms,
- T₈ represents a hydrogen atom or a (-CH₂-CH₂-O-)ₘ₈-H radical in which m8 is an integer of greater than or equal to zero and less than or equal to ten,
- T₉, which is identical to or different from T₈, represents a hydrogen atom or a (-CH₂-CH₂-O-)ₘ₉-H radical in which m9 is an integer of greater than or equal to zero and less than or equal to ten, and
- it being understood that the sum m8 + m9 is greater than 0 and less than or equal to ten;
β) optionally up to 40 mol % of a compound of formula (V'): 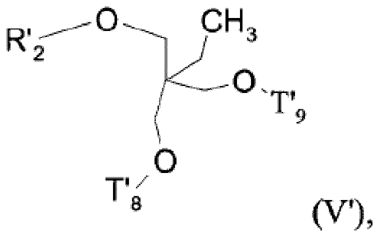 in which:
- R'₂ represents a linear or branched alkyl radical comprising 14 carbon atoms,
- T'₈ represents a hydrogen atom or a (-CH₂-CH₂-O-)ₘ₈-H radical in which m8 is an integer of greater than or equal to zero and less than or equal to ten,
- T'₉, which is identical to or different from T'₈, represents a hydrogen atom or a (-CH₂-CH₂-O-)ₘ₉-H radical in which m9 is an integer of greater than or equal to zero and less than or equal to ten, and
- it being understood that the sum m8 + m9 is greater than 0 and less than or equal to ten; and
γ) optionally up to 10 mol % of a compound of formula (V"): 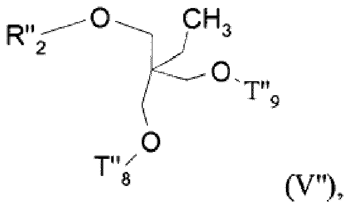 in which:
- R"₂ represents a linear or branched alkyl radical comprising 16 carbon atoms,
- T"₈ represents a hydrogen atom or a (-CH₂-CH₂-O-)ₘ₈-H radical in which m8 is an integer of greater than or equal to zero and less than or equal to ten,
- T"₉, which is identical to or different from T"₈, represents a hydrogen atom or a (-CH₂-CH₂-O-)ₘ₉-H radical in which m9 is an integer of greater than or equal to zero and less than or equal to ten, and
- it being understood that the sum m8 + m9 is greater than 0 and less than or equal to ten.

3. Active composition (C) as defined in either claim 1 or claim 2, additionally comprising:
4) up to 5 mol % of a composition (C_{VI}) comprising, per 100 mol %:
α) from 60 mol % to 100 mol % of a compound of formula (VI):
R2-(-CH₂-CH₂-O-)ₘ₁₀-H (VI),
in which R2 represents a linear or branched alkyl radical comprising 12 carbon atoms and m10 is an integer of greater than or equal to zero and less than or equal to ten;
β) optionally up to 40 mol % of a compound of formula (VI'):
R'₂-(-CH₂-CH₂-O-)m₁₀-H (VI'),
in which R'₂ represents a linear or branched alkyl radical comprising 14 carbon atoms and m10 is an integer of greater than or equal to zero and less than or equal to ten; and
γ) optionally up to 10 mol % of a compound of formula (VI"):
R"₂-(-CH₂-CH₂-O-)ₘ₁₀-H (VI"),
in which R"₂ represents a linear or branched alkyl radical comprising 16 carbon atoms and m10 is an integer of greater than or equal to zero and less than or equal to ten.

4. Surfactant composition (C) as defined in any of claims 1 to 3, **characterised in that** it comprises:
1) a proportion of greater than or equal to 20 mol % and of less than or equal to 50 mol % of a composition (C_{II}) as defined above;
2) a proportion of greater than or equal to 50 mol % and of less than or equal to 80 mol % of a composition (C_{III}) as defined above.

5. Surfactant composition (C) as defined in any of claims 1 to 4, **characterised in that**:
1) said composition (C_{II}) comprises, per 100 mol %:
α) from 60 mol % to 80 mol % of the compound of formula (II),
β) from 15 mol % to 30 mol % of the compound of formula (II'), and
γ) up to 10 mol % of the compound of formula (II"), and
2) said composition (C_{III}) comprises, per 100 mol %:
α) from 60 mol % to 80 mol % of the compound of formula (III), of its isomer of formula (IV) or of the mixture of these isomers,
β) from 15 mol % to 30 mol % of the compound of formula (III'), of its isomer of formula (IV') or of the mixture of these isomers, and
γ) up to 10 mol % of the compound of formula (III"), of its isomer of formula (IV") or of the mixture of these isomers.

6. Process for preparing the surfactant composition (C) as defined in any of claims 1 to 5, **characterised in that** it comprises the following successive stages:
**a stage a)** of reacting a mixture of alcohols comprising, per 100 mol %:
from 60 mol % to 100 mol % of a compound of formula (VII),
R2-O-H (VII),
in which R2 represents a linear or branched alkyl radical comprising 12 carbon atoms; optionally up to 40 mol % of a compound of formula (VII'):
R'₂-O-H (VII'),
in which R'₂ represents a linear or branched alkyl radical comprising 14 carbon atoms, and
optionally up to 10 mol % of a compound of formula (VII"):
R"₂-O-H (VII"),
in which R"₂ represents a linear or branched alkyl radical comprising 16 carbon atoms;
with an excess of 3-(hydroxymethyl)-3-ethyloxetane of formula (VIII): 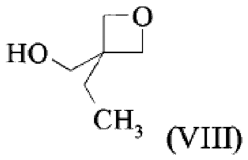 in order to form a composition (C') comprising:
α) the compound of formula (IX): 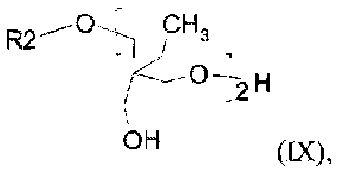 in which R2 is as defined above;
β) optionally the compound of formula (IX'): 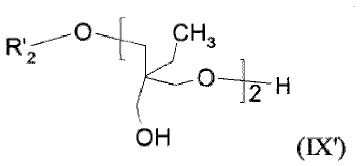 in which R'₂ is as defined above;
γ) optionally the compound of formula (IX"): 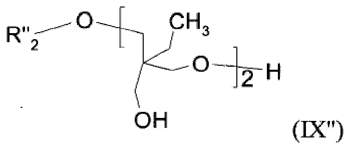 in which R"₂ is as defined above;
δ) the compound of formula (X): 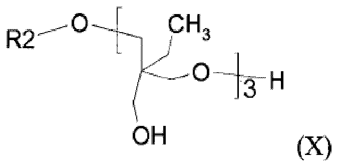 or its isomer of formula (XI): 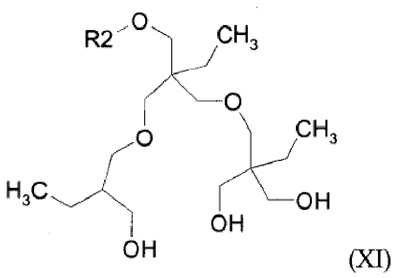 or a mixture of these two isomers;
in which compounds of formulae (X) and (XI) R2 is as defined above;
ε) optionally the compound of formula (X'): 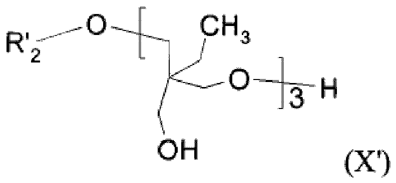 or its isomer of formula (XI'): 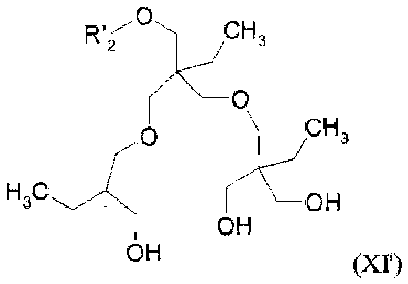 or a mixture of these two isomers;
in which compounds of formulae (X') and (XI') R'₂ is as defined above;
ζ) optionally the compound of formula (X"): 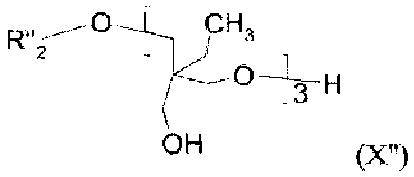 or its isomer of formula (XI"): 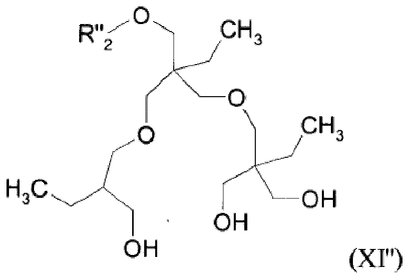 or a mixture of these two isomers;
in which compounds of formulae (X") and (XI") R"₂ is as defined above;
**a stage b)** of reacting said composition (C') with ethylene oxide of formula (XII): 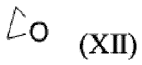 in order to form said surfactant composition (C) as defined above.

7. Composition (C'), which is an intermediate of the process as defined in claim 6.

8. Compounds of formulae (IX), (X) and (XI), which are intermediates of the process as defined in claim 6.

9. Use of the surfactant composition (C) as defined in any of claims 1 to 5, as an emulsifying agent of the oil-in-water (O/W) type.

10. Use as defined in claim 9 of the surfactant composition (C) as an inverting agent for a self-invertible inverse latex, alone or as a mixture with other emulsifying agents which are capable of and intended to prepare an emulsifying system (S₂) of the oil in water (O/W) type.
